# EUROPEAN PATENT APPLICATION

(11) **EP 4 740 885 A1**
(43) Date of publication of application: **13.05.2026**
(21) Application number: 25212328.6
(22) Date of filing: 30.10.2025
(51) Int. Cl.: A61B 34/10, A61B 34/20, A61B 34/30, A61B 34/00, A61B 34/32

(54) **TECHNIQUES FOR ADAPTIVE REORIENTATION OF A ROBOTIC SURGICAL TOOL**

(30) Priority: 07.11.2024 US 202463717640 P
(71) Applicant: MAKO Surgical Corp., Weston, FL 33331 (US)
(72) Inventor: Khurana, Rishabh, Fort Lauderdale, 33301 (US)
(74) Representative: Schott, Jakob Valentin

(57) **Abstract**

Robotic surgical systems and methods involve a tool to manipulate a surgical site and a manipulator to support and move the tool. Controller(s) control the manipulator to facilitate movement of the tool along a tool path for treating the surgical site. During movement of the tool along the tool path, the controller(s) detect a reorientation of the tool and record a location at which the reorientation occurred. The controller(s) generate virtual constraint(s) which cause the manipulator to reorient the tool in response to the tool revisiting the recorded location. In some instances, the controller(s) predictively detect, relative to the tool path, the location at which the tool should be re-orientated, and in response to the tool reaching the predictively detected location, the controller(s) utilize the virtual constraint(s) to cause the manipulator to re-orient the tool.

## Description

### CROSS-REFERENCE TO RELATED APPLICATIONS

The subject application claims benefit of and priority to United States Provisional Patent App. No. 63/717,640, filed November 7, 2024, the entire contents of which is hereby incorporated by reference.

### BACKGROUND

Recently, medical practitioners have found benefit in using robotic systems to perform surgical procedures. Such systems provide robotic control of a surgical tool that moves along a tool path to manipulate a surgical site. Quite commonly, a surgeon may need to manually re-orient the surgical tool as the tool moves along the tool path. For example, the surgeon may manually re-orient the surgical tool to avoid an obstacle or sensitive area at the surgical site. Once the reoriented tool passes the obstacle or sensitive area, the surgeon commonly must re-orient the tool back to its original pose. To make matters more challenging, in some cases, the surgeon may need to repeatedly pass the surgical tool near the obstacle or sensitive area, e.g., in attempt to remove all material from a target region. In such cases, the surgeon may need to execute re-orientations or back-and-forth re-orientations of the surgical tool each time the tool approaches the obstruction. Moreover, tool re-orientations may be needed for surgeon ergonomics, not necessarily for avoiding obstructions. For instance, the surgeon may wish to hold the tool during tool movement but may need to repeatedly re-orient the tool when the tool traverses curved portions of the path to maintain comfortable wrist positioning.

Regardless of the source, such repeated re-orientations can be cumbersome on the surgeon, cause hand or arm fatigue, and create delays in the surgical procedure. Moreover, such conventional techniques force the surgeon to execute the re-orientations under time-pressure e.g., because the tool is moving. These time-pressures can cause surgeon anxiety and potentially result in human error.

### SUMMARY

In a first aspect, a robotic surgical system is provided, comprising: a manipulator configured to support and move a tool along a tool path to manipulate a surgical site; and one or more controllers configured to automatically re-orient the tool at a location along the tool path, the location being identified based on user input defining the location.

In a second aspect, a robotic surgical system is provided, comprising: a manipulator configured to support and move a tool along a tool path to manipulate a surgical site; and one or more controllers configured to automatically re-orient the tool at a location along the tool path, the location being predictively identified by the one or more controllers.

In a third aspect, a robotic surgical system is provided, comprising: a tool configured to manipulate a surgical site; a manipulator configured to support and move the tool; and one or more controllers configured to: control the manipulator to facilitate movement of the tool along a tool path for treating the surgical site; during movement of the tool along the tool path, detect a reorientation of the tool and record a location at which the reorientation occurred; and generate one or more virtual constraints that are configured to cause the manipulator to reorient the tool in response to the tool revisiting the recorded location.

In a fourth aspect, a robotic surgical system is provided, comprising: a tool configured to manipulate a surgical site; a manipulator configured to support and move the tool; and one or more controllers configured to: obtain a tool path defined relative to the surgical site; predictively detect, relative to the tool path, a location at which the tool should be re-orientated; generate one or more virtual constraints that are configured to cause the manipulator to reorient the tool at the predictively detected location; and control the manipulator to facilitate movement of the tool along the tool path for treating the surgical site, and in response to the tool reaching the predictively detected location, utilize the one or more virtual constraints to cause the manipulator to re-orient the tool.

In a fifth aspect, a robotic surgical system is provided, comprising: a tool configured to manipulate a surgical site; a manipulator configured to support and move the tool; and one or more controllers configured to: control the manipulator to facilitate movement of the tool along a tool path for treating the surgical site; during movement of the tool along the tool path, detect a reorientation of the tool and record a location at which the reorientation occurred; and generate feedback that conveys that a re-orientation is needed, wherein the feedback is configured to be delivered in response to the tool revisiting the recorded location.

In a sixth aspect, a robotic surgical system is provided, comprising: a tool configured to manipulate a surgical site; a manipulator configured to support and move the tool; and one or more controllers configured to: obtain a tool path defined relative to the surgical site; predictively detect, relative to the tool path, a location at which the tool should be re-orientated; and generate feedback that conveys that a re-orientation is needed, wherein the feedback is configured to be delivered in response to the tool reaching the predictively detected location.

In a seventh aspect, a robotic surgical system is provided, comprising: a tool configured to manipulate a surgical site; a manipulator configured to support and move the tool along a predetermined tool path; a user input device, and one or more controllers configured to: receive input from the input device that identifies one or more locations relative to the predetermined tool path, wherein the one or more locations signify where the user desires a reorientation of the tool to occur; generate one or more virtual constraints that are configured to cause the manipulator to reorient the tool in response to the tool reaching each of the one or more identified locations; control the manipulator to facilitate movement of the tool along the predetermined tool path for treating the surgical site and utilize the one or more virtual constraints to automatically reorient the tool in response to the tool reaching each of the one or more identified locations.

A method of operating the robotic surgical system of any of the above aspects is provided. Any of the aspects can be combined in part or in whole.

Any of the aspects can be combined with any of the following implementations, which can be recited in part or in whole. The controller(s) can generate at least one virtual constraint at the recorded location. The controller(s) can generate at least one virtual constraint relative to the tool. The robotic surgical system can include a navigation system to track an anatomy of a patient that includes the surgical site. The controller(s) can register the virtual constraint(s) to the tracked anatomy. The controller(s) can customize a feature of the virtual constraint(s). The customized feature can include one or more of: a location of the virtual constraint(s), a geometry of the virtual constraint(s), and/or a stiffness/damping parameter of the virtual constraint(s). Customization can be based on the recorded location relative to the tool path. The robotic surgical system can include a camera that is configured to detect an obstacle or sensitive area at the recorded location. The camera can be attached to the manipulator or tool or located remote from the manipulator or tool. The controller(s) can customize a feature of the virtual constraint(s) based on the detected obstacle or sensitive area. The controller(s) can detect a parameter of the tool during, or after, occurrence of the reorientation. The detected parameter of the tool can include one or more of: a displacement of the tool, a direction of force applied to the tool, a magnitude of force applied to the tool, a velocity of the tool, and an acceleration of the tool. The controller(s) can customize a feature of the virtual constraint(s) based on the detected parameter of the tool. The controller(s) can generate the virtual constraint(s) in a manner that causes the manipulator to reorient the tool with a magnitude and direction that emulates the detected reorientation. The controller(s) can generate the virtual constraint(s) in a manner that accounts for one or more of: a workspace limit of the manipulator; joint limits of the manipulator; and singularities of the manipulator. The controller(s) can compare the detected parameter of the tool to a threshold value; and in response to the detected parameter satisfying the threshold value, determine that the reorientation of the tool is an intended manual reorientation responsive to an external force applied to the tool by a user. The tool can include a user interface configured to receive an input to initiate re-orientation of the tool. The controller(s) can detect the reorientation in response to the receipt of the input from the user interface. The virtual constraint(s) can comprise a virtual mesh, which optionally can be defined at the recorded location and/or relative to the tool. The controller(s) can cause the manipulator to reorient the tool in response to interaction between the tool and the virtual mesh. The virtual constraint(s) can comprise at least one stereotactic interaction, which optionally can be defined at the recorded location and/or relative to the tool. The controller(s) can cause the manipulator to reorient the tool in response to interaction between the at least one stereotactic interaction feature and the virtual mesh, or in response to interaction between two or more stereotactic interaction features. The tool can comprise a tool shaft and the at least one stereotactic interaction feature can be defined relative to the tool shaft. The virtual constraint(s) can comprise an attractive force or a repulsive force, which can optionally be defined in proximity to the recorded location. The controller(s) can cause the manipulator to reorient the tool in response to the tool experiencing the attractive force or the repulsive force or entering a zone defining the attractive force or the repulsive force. The tool can be in an original pose prior to being reoriented by the virtual constraint(s). The controller(s) can reorient the tool back to the original pose once the tool leaves the recorded location. The controller(s) can modify a feature of the virtual constraint(s) in response to detection of a second reorientation of the tool that occurs proximate to the recorded location. Any of the features of the controller(s) can be implemented automatically, including automatic movement of the tool along the tool path, automatic generating of the virtual constraint(s), and automatically causing the manipulator to reorient the tool with the virtual constraint(s). The controller(s) can dynamically remove or deactivate the virtual constraint(s) in response to detection of any condition, such as completion of a surgical step or determining that the tool no longer needs to pass near the recorded location.

The controller(s) can predictively detect the location based on identification of the obstacle or sensitive area by the camera. The controller(s) can predictively detect the location based on detection of one or more prior re-orientations of the tool. The controller(s) can predictively detect the location based on detection of one or more prior detected parameters of the tool. The controller(s) can predictively detect the location by utilizing a machine learning model that is trained on prior surgical plan data and prior manipulator data, such as tool path data, re-orientation data, implant data, kinematic data, and camera data. The controller(s) can predictively customize a feature of the virtual constraint(s). The predictively customized feature includes one or more of: a location of the virtual constraint(s), a geometry of the virtual constraint(s), and/or a stiffness/damping parameter of the virtual constraint(s).

The controller(s) can generate feedback that conveys that a re-orientation is needed at a recorded or predictively detected location. The feedback can be delivered in response to the tool revisiting the recorded location. The feedback can be haptic feedback provided by the manipulator. The feedback can be visual feedback provided by a light indicator provided on the tool. The feedback can be visual and provided on a display that also presents a representation of the tool, the tool path, and the surgical site. The feedback can include a visual identification of the location relative to the representation of the tool path on the display. The feedback can include a text message or verbal warning that conveys that the re-orientation is needed.

### BRIEF DESCRIPTION OF THE DRAWINGS

Advantages of the present invention will be readily appreciated as the same becomes better understood by reference to the following detailed description when considered in connection with the accompanying drawings wherein:
Figure 1 is a perspective view of a robotic system including a manipulator, according to one implementation;
Figure 2 is a schematic view of controller(s) of the robotic system, according to one implementation;
Figure 3 is an illustration of a surgical tool moving along a tool path, according to one implementation;
Figure 4 is an illustration of a tool path for treating an anatomy of a patient, according to one implementation;
Figure 5 is a flowchart of a method of facilitating reorientation of a surgical tool, according to one implementation;
Figure 6 is an illustration of a reorientation of a surgical tool while the surgical tool moves along a tool path during operation of the manipulator in a semi-autonomous, automatic, or guided-manual mode, according to one implementation;
Figure 7 is an illustration of a reorientation of a surgical tool during operation of the manipulator in a manual mode, according to one implementation;
Figure 8 is an illustration of a virtual mesh generated during the method of facilitating reorientation of a surgical tool, according to one implementation;
Figure 9 is an illustration of a stereotactic interaction feature generated during the method of facilitating reorientation of a surgical tool, according to one implementation;
Figure 10 is an illustration of a particle guide generated during the method of facilitating reorientation of a surgical tool, according to one implementation;
Figures 11A-11B are illustrations of a configuration wherein the controller(s) modify a virtual constraint by reducing a size of the virtual constraint, according to one implementation;
Figures 12A-12B are illustrations of a configuration wherein the controller(s) modify a virtual constraint by increasing a size of the virtual constraint, according to one implementation;
Figures 13A-13B are illustrations of a configuration wherein the controller(s) merge more than one virtual constraint into an aggregate virtual constraint, according to one implementation; and
Figures 14A-14B are illustrations of a configuration wherein the controller(s) automatically generate a virtual constraint, according to one implementation.

### DETAILED DESCRIPTION

### I. SYSTEM OVERVIEW

With reference to the Figures, wherein like numerals indicate like or corresponding parts throughout the several views, a surgical system 10 (hereinafter "system") and method for operating the system 10 are described herein and shown throughout the accompanying Figures.

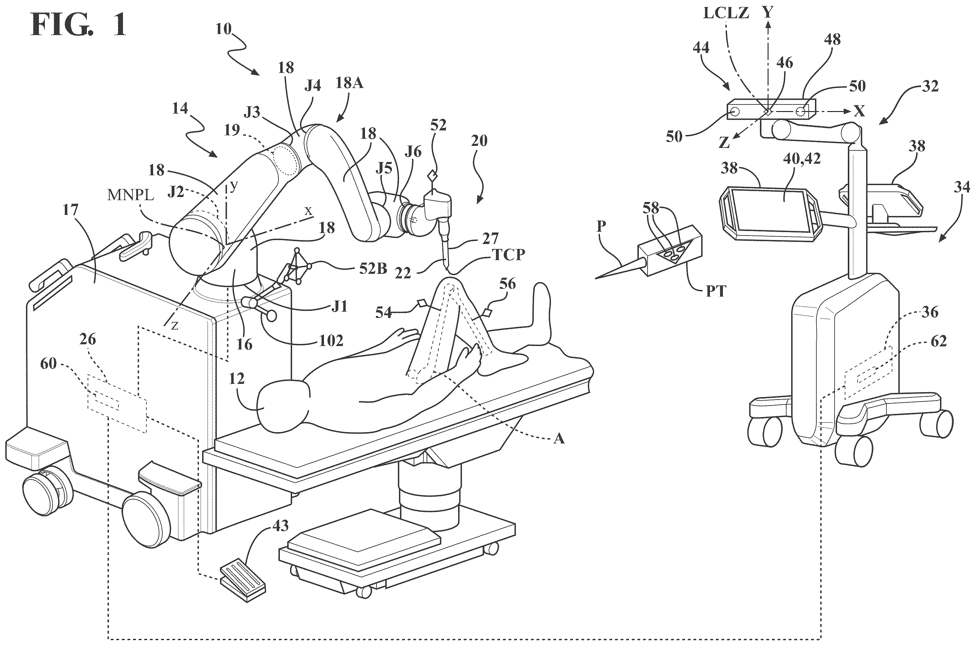

As shown in FIG. 1, the system 10 is a robotic surgical system for treating an anatomy (surgical site) of a patient 12, such as bone or soft tissue. In FIG. 1, the patient 12 is undergoing a surgical procedure. The anatomy A in FIG. 1 includes a femur and tibia of the patient 12. The surgical procedure may involve tissue removal or treatment. The robotic surgical system 10 described herein may be utilized for treating any anatomical structure(s), for example, such as joints, including knee joints, hip joints, shoulder joints, ankles joints, or any other bone structure(s) not described herein. The robotic surgical system 10 can be used to perform any type of procedure, including any spinal procedure, partial knee arthroplasty, total knee arthroplasty, total hip arthroplasty, anatomical shoulder arthroplasty, reverse shoulder arthroplasty, fracture repair surgery, osteotomies, and the like. Similarly, the techniques and methods described herein can be used with any type of robotic system and for any procedure.

The system 10 includes a manipulator 14, which may also be referred to as a robotic manipulator. In one example, the manipulator 14 has a base 16 and plurality of links 18. The plurality of links 18 may be commonly referred to as a robotic arm 18A. In some instances, the manipulator 14 may include more than one robotic arm 18A. A manipulator cart 17 supports the manipulator 14 such that the manipulator 14 is fixed to the manipulator cart 17. The links 18 collectively form one or more arms of the manipulator 14. The manipulator 14 may have a serial arm configuration (as shown in FIG. 1) or a parallel arm configuration. In other examples, more than one manipulator 14 may be utilized in a multiple arm configuration. The manipulator 14 comprises a plurality of joints (J) and a plurality of joint encoders 19 located at the joints (J) for determining position data of the joints (J). For simplicity, one joint encoder 19 is illustrated in FIG. 1, although it is to be appreciated that the other joint encoders 19 may be similarly illustrated. The manipulator 14 according to one example, has six joints (J1-J6) implementing at least six-degrees of freedom (DOF) for the manipulator 14. However, the manipulator 14 may have any number of degrees of freedom and may have any suitable number of joints (J) and redundant joints (J). In one example, each of the joints (J) of the manipulator 14 are actively driven and may be motorized joints (J). In other examples, each of the joints (J) may be passively driven. In still other examples, the joints (J) may include a combination of actively driven joints (J) and passively driven joints (J).

The base 16 of the manipulator 14 is generally a portion of the manipulator 14 that is stationary during usage thereby providing a fixed reference coordinate system (i.e., a virtual zero pose) for other components of the manipulator 14 or the system 10 in general. Generally, the origin of a base coordinate system is defined at the fixed reference of the base 16. The base coordinate system may be referred to herein as a manipulator coordinate system MNPL and the robotic arm 18A is configured to support and move an end effector coupled to the robotic arm 18A in the manipulator coordinate system MNPL. The fixed reference point of the base 16 may be defined with respect to any suitable portion of the manipulator 14, such as one or more of the links 18. Alternatively, or additionally, the fixed reference point of the base 16 may be defined with respect to the manipulator cart 17, such as where the manipulator 14 is physically attached to the cart 17. In one example, the fixed reference point of the base 16 is defined at an intersection of the axes of joints J1 and J2. Thus, although joints J1 and J2 are moving components in reality, the intersection of the axes of joints J1 and J2 is nevertheless a virtual fixed reference point, which does not move in the manipulator coordinate system MNPL. The manipulator 14 and/or manipulator cart 17 house a manipulator computer 26, or other type of control unit. In other examples, the manipulator 14 can be a hand-held robotic device where the base 16 is a portion of the surgical tool 22 that is grasped by hand and the surgical tool 22 is attached to the grasped portion by one or more actuators that move the surgical tool 22 in one or more degrees of freedom relative to grasped portion.

The system 10 may include an end effector 20 coupled to the robotic arm 18A. The end effector 20 may include any end effector suitable for a surgical procedure. In some instances, the end effector 20 may include a surgical tool 22 such that the surgical tool 22 is supported by the robotic arm 18A. The surgical tool 22 may be any instrument for manipulating the anatomy A of a patient, such as a saw, a cutting burr, a router, a reamer, an impactor, an ultrasonic aspirator, a probe, a scalpel, a trocar, a cutting tool, a drill, a dilator, a screwdriver, an intervertebral inserter, a distractor, an abrator, a discectomy tool, or the like. In the instance of FIG. 1, the end effector 20 includes a surgical tool 22, which is illustrated as a drill device. Additionally, or alternatively, the end effector 20 may include an accessory and/or energy applicator, such as a saw blade, a cutting burr, a router, a reamer, an impactor, an ultrasonic aspirator, a probe, a scalpel, a trocar, a cutting tool, a drill, a dilator, a screwdriver, an intervertebral inserter, a distractor, an abrator, a discectomy tool, or the like. The accessory and energy applicator may be integrated or separately attached to the end effector 20. In some instances, the end effector 20 may include a shaft 27 and the energy applicator may be located on an end of the shaft 27. The end effector 20 may also include a cutting guide. As shown in FIG. 1, the end effector 20 may include a tool holder, which may support any of the surgical tools 22 described above. The tool holder may be a guide tube for supporting a surgical tool 22. The surgical tool 22 may be temporarily affixed to the guide tube and/or slidable within the guide tube. The guide tube may be the guide tube further described in U.S. Provisional Patent Application No. 63/612,011, entitled, "Magnetic Spine Registration Tool", which is incorporated herein by reference. Additionally, the guide tube may be like that described in U.S. Provisional Patent Application No. 63/454,346, entitled, "Anti-Skiving Guide Tube And Surgical System Including The Same", which is incorporated herein by reference. Additionally, or alternatively, the surgical tools 22 can be actively driven or motorized by the robotic manipulator 14. The surgical tools 22 can be hand-held and selectively coupled to the robotic manipulator 14.

The system 10 may include one or more tool trackers. The tool tracker may be temporarily coupled to the end effector 20. For example, the tool tracker may be the trackable array described in U.S. Patent App. Pub. No. 2022/0134569, entitled, "Robotic Surgical System With Motorized Movement To A Starting Pose For A Registration Or Calibration Routine," the disclosure of which is hereby incorporated by reference, or such as the end effector tracker described in U.S. Patent No. 10,350,012, entitled, "Method And Apparatus For Controlling A Haptic Device," the disclosure of which is hereby incorporated by reference, or such as the tool tracker in U.S. Provisional Patent Application No. 63/612,011, entitled, "Magnetic Spine Registration Tool", which is incorporated herein by reference. In other examples, the tool tracker may be attachable to or detachable from the end effector 20 and/or attachable to or detachable from any other component of the manipulator 14, such as one or more links of the robotic arm 18A, e.g. a distal-most link of the manipulator (J6). For instance, the tool tracker may include similar components as the tracker assembly described in U.S. Patent App. Pub. No. 2023/0277256, entitled, "Robotic System Including a Link Tracker," the disclosure of which is hereby incorporated by reference, for attaching the tool tracker to the end effector 20 or any other component of the manipulator 14. For instance, the tool tracker may be attached/detached to the end effector 20 or any other component of the manipulator 14 using a spring-biased latch, a magnetic connection, a snap-fit connection using flexible elements, or the like. In other examples, the tool tracker may be temporarily coupled to the end effector 20 via a component of the end effector 20. For example, in instances where the end effector 20 includes the surgical tool 22, the tool tracker may be coupled to the end effector 20 via the surgical tool 22. As another example, in instances where the end effector 20 includes a guide tube, the tool tracker may be coupled to the end effector 20 via the guide tube. Additionally, the system 10 may include more than one tool tracker. For example, in instances where the end effector 20 includes the guide tube configured to support a surgical tool 22 temporarily affixed to the guide tube, a first tool tracker may be coupled to the guide tube, and a second tool tracker may be coupled to the surgical tool 22.

The tool tracker may be coupled to the end effector 20 such that a relationship between the tool tracker and the end effector 20 may be determinable. For example, the tool tracker may include a reference surface configured to abut the end effector 20, such as the reference surface described in U.S. Provisional Patent Application No. 63/612,011, entitled, "Magnetic Spine Registration Tool", which is incorporated herein by reference. Contact between the reference surface and the end effector 20 may indicate that the tool tracker is properly coupled to the end effector 20 such that a location of the tool tracker relative to the end effector 20 is fixed and that a relationship between the tool tracker and the end effector 20 is determinable.

The tool tracker may include one or more fiducial markers FM. In some instances, the fiducial markers FM may be coupled to or integrally formed with or manually coupled to the end effector 20 and/or a component of the manipulator 14. The fiducial markers FM may include any suitable shape. For example, the fiducial markers FM may include a cuboidal or elliptical shape. The fiducial markers FM may be active or passive tracking elements.

As shown in FIG. 1, the system 10 further includes a navigation system 32. One example of the navigation system 32 is described in U.S. Patent No. 9,008,757, filed on Sep. 24, 2013, entitled, "Navigation System Including Optical and Non-Optical Sensors," hereby incorporated by reference. The navigation system 32 may track movement of various objects. Such objects include, for example, the manipulator 14, the end effector 20, and/or the anatomy A. The navigation system 32 tracks these objects to gather state information of one or more of the objects with respect to a (navigation) localizer coordinate system LCLZ. Coordinates in the localizer coordinate system LCLZ may be transformed to the manipulator coordinate system MNPL, and/or vice-versa, using transformation and registration techniques described in U.S. Provisional Patent Application No. 63/552,897, entitled "Systems and Method for Image Based Registration and Calibration," U.S. Provisional Patent Application No. 63/612,011, entitled, "Magnetic Spine Registration Tool", and U.S. Patent Application No. 17/513,324, entitled "Robotic Surgical System with Motorized Movement to a Starting Pose for a Registration or Calibration Routine", which are incorporated herein by reference.

The navigation system 32 can include a cart assembly 34 that houses a navigation computer 36, and/or other types of control units. A navigation interface is in operative communication with the navigation computer 36. The navigation interface includes one or more displays 38. The navigation system 32 is capable of displaying a graphical representation of the relative states of the tracked objects to an operator using the one or more displays 38.

The navigation system 32 may depict a visual representation of the anatomy A and the manipulator 14, and/or end effector 20 for visual guidance of any of the techniques described. The visual representation may be real (camera) images, virtual representations (e.g., 2D and/or 3D computer models), or any combination thereof. The visual representation can be presented on any display viewable to the operator, such as the displays 38 of the navigation system 32, a display of a head-mounted device, or the like. The representations may be augmented reality, mixed reality, or virtual reality. In some implementations, the display may be configured as an extended reality device configured to execute any of the graphical functions described herein. The extended reality device can be implemented by a hand-held device (e.g., tablet or smart phone) or a head-mounted device. The extended reality device may be configured to superimpose, overlay, or combine any of the described computer-generated graphics with real-world views to implement an extended reality, augmented reality, and/or mixed reality experience for the operator. The real-world views may be those acquired directly by the eyes of the operator or may be a real-world video stream captured by one or more cameras of the extended reality device. When a head-mounted device is utilized, the head-mounted device may comprise a transparent lens or one or more display screens positioned directly in front of the eyes of the operator to display the computer-generated graphics relative to the real-world views."

The navigation system 32 also includes a navigation localizer 44 (hereinafter "localizer") coupled to the navigation computer 36. In one example, the localizer 44 is an optical localizer and includes a camera unit 46. The camera unit 46 has an outer casing 48 that houses one or more optical sensors 50.

The navigation system 32 may include one or more trackers, which may be tracked by the localizer 44. In one example, the trackers include the tool tracker, a pointer tracker PT, one or more manipulator trackers 52, and/or one or more patient trackers 54, 56. In the illustrated example of FIG. 1, the manipulator tracker 52 is attached to a distal flange of the robotic arm 18A. The manipulator tracker 52 may be affixed to any suitable component of the manipulator 14, in addition to, or other than the surgical tool, such as the base 16 (i.e., tracker 52B), or any one or more links 18 or joints J of the manipulator 14. Additionally, or alternatively, the manipulator tracker 52 may be secured to a surgical drape or drape assembly, as described in U.S. Patent App. Pub. No. 2023/0277256, entitled, "Robotic System Including a Link Tracker," the disclosure of which is hereby incorporated by reference. For instance, the manipulator tracker 52 may be secured to a surgical drape or drape assembly via an elastic band or snap ring. The patient trackers may be affixed to the anatomy A of the patient 12. In the illustrated example of FIG. 1, the first patient tracker 54 is firmly affixed to the femur of the patient 12, and the second patient tracker 56 is firmly affixed to the tibia of the patient 12. In this example, the patient trackers 54, 56 are firmly affixed to sections of bone. The pointer tracker PT is firmly affixed to a pointer P used for registering the anatomy A to the localizer coordinate system LCLZ. Those skilled in the art appreciate that the trackers described herein may be fixed to their respective components in any suitable manner.

As shown in FIG. 1, the base tracker 52B may be coupled to the cart 17 by an adjustable support arm 102. As shown, the base tracker 52B may be attached to one end of an adjustable support arm 102 and the adjustable support arm 102 may be attached at the other end to the cart 17. The adjustable support arm 102 can be positioned and locked to place the base tracker 52B in a fixed position relative to the cart 17. An example of a base tracker 52B coupled to an adjustable support arm can be like that described in U.S. Patent App. No. 17/513,324, entitled, "Robotic Surgical System With Motorized Movement To A Starting Pose For A Registration Or Calibration Routine", or U.S. Patent App. No. 18/198,938, entitled, "Robotic System With Improved Configurations For Base Tracker", the entire contents of which are hereby incorporated by reference in their entirety . Alternatively, or additionally, a base tracker 52B may be coupled to the robotic arm 18A and may be moveable with the robotic arm 18A. For instance, the base tracker 52B may include a plurality of (active or passive) tracking elements located on any number of links 18 of the manipulator 14. In this case, the base tracker 52B is formed of a tracking geometry from the various tracking elements, which move with movement of the robotic arm 18A. An example of a base tracker 52B formed by optical markers located on the links 18 may be like that described in US Patent App. No. 18/115964, entitled, "Robotic System with Link Tracker", the entire contents of which is hereby incorporated by reference in its entirety. Alternatively, or additionally, the base tracker 52B may be secured to a surgical drape or drape assembly, as described in U.S. Patent App. Pub. No. 2023/0277256, entitled, "Robotic System Including A Link Tracker," the disclosure of which is hereby incorporated by reference. For instance, the base tracker 52B may be secured to a surgical drape or drape assembly via an elastic band or snap ring.

When optical localization is utilized, however, one or more of the trackers may include active markers 58. The active markers 58 may include light emitting diodes (LEDs). Alternatively, the trackers described herein may have passive markers, such as reflectors, which reflect light emitted from the camera unit 46. Other suitable markers not specifically described herein may be utilized.

The localizer 44 tracks the trackers to determine a state of one or more of the trackers which correspond respectively to the state of the object attached thereto. The localizer 44 provides the state of the trackers to the navigation computer 36. In one example, the navigation computer 36 determines and communicates the state of the trackers to the manipulator computer 26. As used herein, the state of an object includes, but is not limited to, data that defines the position and/or orientation of the tracked object or equivalents/derivatives of the position and/or orientation. For example, the state may be a pose of the object, and may include linear velocity data, and/or angular velocity data, and the like.

Although one example of the navigation system 32 is shown in the Figures, the navigation system 32 may have any other suitable configuration for tracking the manipulator 14 and the patient 12. The illustrated tracker configuration is provided merely as one example for tracking objects within the operating space. Any number of trackers may be utilized and may be located in positions or on objects other than shown. In other examples, such as described below, the localizer 44 may detect objects absent any trackers affixed to objects.

In one example, the navigation system 32 and/or localizer 44 are ultrasound-based. For example, the navigation system 32 may comprise an ultrasound imaging device coupled to the navigation computer 36. The ultrasound imaging device may be robotically controlled or may be hand-held. The ultrasound imaging device images any of the aforementioned objects, e.g., the manipulator 14 and the patient 12, and generates state signals to the controller 30 based on the ultrasound images. The ultrasound images may be of any ultrasound imaging modality. The navigation computer 36 may process the images in near real-time to determine states of the objects. Ultrasound tracking can be performed absent the use of trackers affixed to the objects being tracked. The ultrasound imaging device may have any suitable configuration and may be different than the camera unit 46 as shown in FIG. 1. One example of an ultrasound tracking system can be like that described in U.S. patent application Ser. No. 15/999,152, filed Aug. 16, 2018, entitled "Ultrasound Bone Registration With Learning-Based Segmentation And Sound Speed Calibration," the entire contents of which are incorporated by reference herein.

In another example, the navigation system 32 and/or localizer 44 are radio frequency (RF)-based. For example, the navigation system 32 may comprise an RF transceiver coupled to the navigation computer 36. The manipulator 14 and the patient 12 may comprise RF emitters or transponders attached thereto. The RF emitters or transponders may be passive or actively energized. The RF transceiver transmits an RF tracking signal and generates state signals to the controller 30 based on RF signals received from the RF emitters. The navigation computer 36 and/or the controller 30 may analyze the received RF signals to associate relative states thereto. The RF signals may be of any suitable frequency. The RF transceiver may be positioned at any suitable location to track the objects using RF signals effectively. Furthermore, the RF emitters or transponders may have any suitable structural configuration that may be much different than the trackers as shown in FIG. 1.

In yet another example, the navigation system 32 and/or localizer 44 are electromagnetically based. For example, the navigation system 32 may comprise an EM transceiver coupled to the navigation computer 36. The manipulator 14 and the patient 12 may comprise EM components attached thereto, such as any suitable magnetic tracker, electro-magnetic tracker, inductive tracker, or the like. The trackers may be passive or actively energized. The EM transceiver generates an EM field and generates state signals to the controller 30 based upon EM signals received from the trackers. The navigation computer 36 and/or the controller 30 may analyze the received EM signals to associate relative states thereto. Again, such navigation system 32 examples may have structural configurations that are different than the navigation system 32 configuration as shown throughout the Figures.

In yet another example, the navigation system 32 and/or localizer 44 utilize a machine vision system which includes a vision camera coupled to the navigation computer 36. The machine vision system can include any suitable type of imaging modality for scanning surfaces, such as structured light, multi-modal (visible/NIR), plenoptic (light field), ultrasound, spectral imaging, LIDAR, photogrammetry, SfM (structure from motion), and the like. The machine vision system may include the vision camera coupled directly to the manipulator 14 or located separately therefrom. For example, the vision camera can be coupled to the camera unit of the navigation system, coupled to a surgical tool/pointer, included in a smartphone/tablet, located in a head-mounted device, or provide in a multi-view camera/boom. The vision camera may scan the target site, end effector, and any physical object in a target space. The physical object may have a geometry represented by virtual object data stored by the navigation computer 36. The detected objects may be tools, obstacles, obstructions, sensitive areas, anatomical features, trackers, or the like. The vision camera and navigation computer 36 are configured to detect the physical objects using image processing techniques such as point-cloud generation, pattern, color, or shape recognition, edge detection, pixel analysis, neutral net or deep learning processing, optical character recognition, barcode detection, and the like. The navigation computer 36 can compare the captured images to the virtual object data to identify and track the objects. The machine vision system may operate in a trackerless manner. A tracker may or may not be coupled to the physical object. If trackers are utilized, the machine vision system may also include infrared detectors for tracking the trackers and comparing tracking data to machine vision data. Again, such navigation system 32 examples may have structural configurations that are different than the navigation system 32 configuration as shown throughout the Figures. Examples of machine vision tracking systems can be like that described in U.S. Patent No. 9,603,665, entitled "Systems and Methods for Establishing Virtual Constraint Boundaries" and/or like that described in U.S. Provisional Patent Application No. 62/698,402, filed Jul. 16, 2018, entitled "Systems and Method for Image Based Registration and Calibration," the entire contents of which are incorporated by reference herein.

The navigation system 32 and/or localizer 44 may have any other suitable components or structure not specifically recited herein. Furthermore, any of the techniques, methods, and/or components described above with respect to the camera-based navigation system 32 shown throughout the Figures may be implemented or provided for any of the other examples of the navigation system 32 described herein. For example, the navigation system 32 may utilize solely inertial tracking or any combination of tracking techniques.

The system 10 may include one or more input/output devices 40, 42, 43. The input/output devices may receive an input from an operator interacting with an input/output device 40, 42, 43. Additionally, as will be described in greater detail below, the input/output devices may output haptic, audible, and/or visual feedback to an operator of the surgical system 10. In FIG. 1, the first and second input/output devices 40, 42 are shown as interactive touchscreen displays and the third input/output device 43 is shown as a footswitch including a user-actuatable foot pedal. In other instances, the input/output devices 40, 42, 43 may be any device for receiving an input from an operator and/or outputting haptic, audible, and/or visual feedback to the operator. For example, the input/output device 40, 42, 43 may include any one or more of a speaker, a display, a smartphone device, a keyboard, a mouse, a remote-control device, a microphone (voice-activation), gesture control devices, head-mounted devices, and the like. Additionally, the input/output devices 40, 42, 43 may be the haptic device described in U.S. Patent No. 10,350,012, entitled, "Method and Apparatus for Controlling a Haptic Device," the disclosure of which is hereby incorporated by reference.

Additionally, it is contemplated that the operator may interact with the input/output devices 40, 42, 43 in any suitable manner and the input/output devices 40, 42, 43 may receive a corresponding input. For example, the operator may interact with the input/output devices 40, 42, 43 by pressing, holding, clicking, double-clicking, releasing, and/or performing any other suitable interaction with an input of the input/output devices 40, 42, 43. In a more specific instance, the operator may interact with the footswitch 43 of FIG. 1 by pressing, holding, clicking, double-clicking, and/or releasing the user-actuatable foot pedal of the footswitch 43. Similarly, the operator may interact with the interactive touchscreen displays 40, 42 of FIG. 1 by pressing, holding, clicking, double-clicking, swiping, and/or releasing a portion of a graphical user interface of the interactive touchscreen displays 40, 42. In instances where the input/output devices 40, 42, 43 include a head-mounted device, the head-mounted device may be configured to receive the input by tracking a gesture, a gaze, and/or a head motion of the operator. The head-mounted device may also include a microphone and/or audio sensor configured to receive voice instructions/commands as the confirmation from the user.

### II. CONTROLLER OVERVIEW

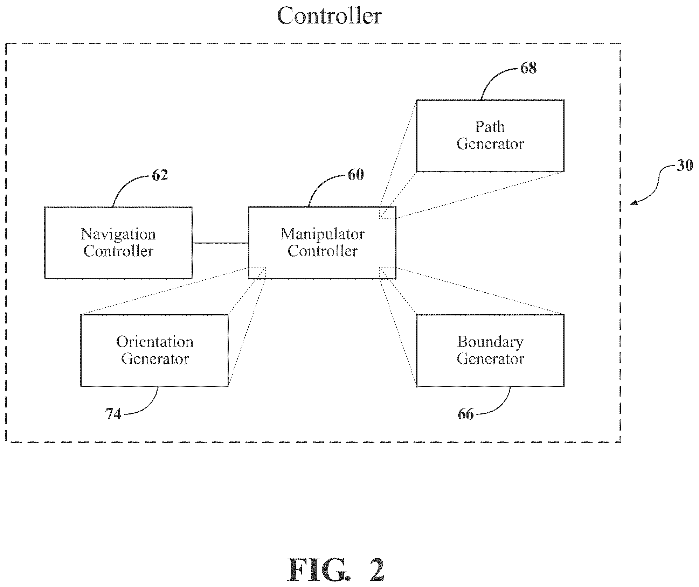

Referring to FIG. 2, the system 10 includes one or more controllers 30 (hereinafter referred to as "controller"). The controller 30 includes software and/or hardware for controlling the manipulator 14. The controller 30 directs the motion of components of the manipulator 14, such as the robotic arm 18A, and controls a pose (position and/or orientation) of the end effector 20 with respect to a coordinate system of the robotic arm 18A. In one example, the coordinate system of the robotic arm 18A is the manipulator coordinate system MNPL, as shown in FIG. 1, and the controller 30 may control the robotic arm 18A to support and move the end effector 20 in the manipulator coordinate system MNPL. The manipulator coordinate system MNPL has an origin located at any suitable pose with respect to the manipulator 14. Axes of the manipulator coordinate system MNPL may be arbitrarily chosen as well. Generally, the origin of the manipulator coordinate system MNPL is defined at the fixed reference point of the base 16. One example of the manipulator coordinate system MNPL is described in U.S. Patent No. 9,119,655, entitled, "Surgical Manipulator Capable of Controlling a Surgical Instrument in Multiple Modes," the disclosure of which is hereby incorporated by reference.

As shown in FIG. 2, the controller 30 further includes software modules. The software modules may be part of a computer program or programs that operate on the manipulator computer 26, navigation computer 36, or a combination thereof, to process data to assist with control of the system 10. The software modules include instructions stored in one or more non-transitory computer readable medium or memory on the manipulator computer 26, navigation computer 36, or a combination thereof, to be executed by one or more processors of the computers 26, 36. Additionally, software modules for prompting and/or communicating with the operator may form part of the program or programs and may include instructions stored in memory on the manipulator computer 26, navigation computer 36, or a combination thereof.

The controller 30 includes a manipulator controller 60 for processing data to direct motion of the manipulator 14. In one example, as shown in FIG. 1, the manipulator controller 60 is implemented on the manipulator computer 26. The manipulator controller 60 may receive and process data from a single source or multiple sources. The controller 30 further includes a navigation controller 62 for communicating the state data relating to the anatomy A to the manipulator 14 to the manipulator controller 60. The manipulator controller 60 receives and processes the state data provided by the navigation controller 62 to direct movement of the manipulator 14. In one example, as shown in FIG. 1, the navigation controller 62 is implemented on the navigation computer 36. The manipulator controller 60 or navigation controller 62 may also communicate states of the patient 12 and manipulator 14 to the operator by displaying a 2D and/or 3D image of the anatomy A and the manipulator 14 on the one or more displays 38. The manipulator computer 26 or navigation computer 36 may also command display of instructions or request information using the display 38 to interact with the operator and for directing the manipulator 14.

The controller 30, including the manipulator controller 60 and navigation controller 62, may be implemented on any suitable device or devices in the system 10, including, but not limited to, the manipulator computer 26, the navigation computer 36, and any combination thereof. As will be described herein, the controller 30 is not limited to one controller, but may include a plurality of controllers for various systems, components, or sub-systems of the surgical system 10. These controllers may be in communication with each other (e.g., directly, or indirectly), and/or with other components of the surgical system 10, such as via physical electrical connections (e.g., a tethered wire harness) and/or via one or more types of wireless communication (e.g., with a WiFi^{™} network, Bluetooth^{®}, a radio network, and the like). Any of the controller 30 may be realized as or with various arrangements of computers, processors, control units, and the like, and may comprise discrete components or may be integrated (e.g., sharing hardware, software, inputs, outputs, and the like). Any of the one or more controllers may implement their respective functionality using hardware-only, software-only, or a combination of hardware and software. Examples of hardware include, but is not limited, single or multi-core processors, CPUs, GPUs, integrated circuits, microchips, or ASICs, digital signal processors, microcontrollers, field programmable gate arrays, systems on a chip, discrete circuitry, and/or other suitable hardware, and the like. The controller(s) may implement software programs, software modules, algorithms, logical rules, look-up tables and other reference data, and various software layers for implementing any of the capabilities described herein. Equivalents of the software and hardware for the controller 30, and peripheral devices connected thereto, are fully contemplated.

### a. Boundary Generator

As shown in FIG. 2, the controller 30 includes a boundary generator 66. The boundary generator 66 is a software module that may be implemented on the manipulator controller 60. Alternatively, the boundary generator 66 may be implemented on any other suitable component of the system 10, such as the navigation controller 62.

The boundary generator 66 may generate virtual constraints that represent the tracked anatomy of the patient 12. Such virtual constraints may include virtual boundaries, virtual meshes, virtual objects, or the like. For example, the boundary generator 66 may generate a virtual mesh indicating a shape, size, and position of a bone of the patient 12.

The boundary generator 66 may generate virtual constraints for constraining the manipulator 14 and/or the end effector 20. Such virtual constraints may include virtual boundaries, virtual meshes, virtual objects, or the like. Such virtual constraints may correspond to any object detected by the navigation system 32. The virtual constraints may be defined with respect to a tracked anatomy of the patient 12. For example, the controller 30 may generate a 3-D bone model registered to one or more patient trackers. The controller 30 may register the virtual constraints to the bone model to fix the virtual constraints relative to the bone model. Additionally, or alternatively, the virtual constraints may be defined with respect to the surgical site and/or the end effector 20.

In some instances, the virtual constraint may be a particle guide. A particle guide may be generated by the boundary generator 66 and the controller 30 may generate a constraint force adapted to attract the surgical tool 22 toward the particle guide or repel away from the particle guide. The constraint force is further described in U.S. Patent Application No. 17/701,989, entitled, "Systems and Methods for Guiding Movement of a Tool", the disclosure of which is hereby incorporated by reference.

In some instances, the virtual constraint may be a stereotactic interaction feature. A stereotactic interaction feature may be generated by the boundary generator 66 and may be defined relative to the end effector 20. For example, the stereotactic interaction feature may be generated at a position attributed to the surgical tool 22. These stereotactic interaction features may include any suitable geometric shape and any suitable size. For instance, the stereotactic interaction features may be point and/or spheres with unique origins and radii. The stereotactic interaction features may be generated relative to the end effector 20 such that the stereotactic interaction features may collide with a virtual constraint instead of the end effector 20. Stereotactic interaction features are further described in U.S. Patent No. 11,944,396, entitled, "Systems and Methods for Controlling Robotic Movement of a Tool Based on a Virtual Boundary", the disclosure of which is hereby incorporated by reference.

A state of the manipulator 14 and/or the end effector 20 may be tracked relative to the virtual constraints. For example, the state of the manipulator 14 and/or the end effector 20 may be tracked to determine whether the surgical tool 22 is interacting with a virtual constraint. In one example, a state of a center point of the end effector 20 and/or surgical tool 22 is measured relative to the virtual constraints for purposes of determining to whether the surgical tool 22 is interacting with a virtual constraint. Furthermore, the boundary generator 66 may generate a virtual representation of the surgical tool 22 to determine whether the surgical tool 22 is interacting with a virtual constraint. Accordingly, the boundary generator 66 may apply a haptic feedback force to the manipulator 14, or more specifically, the end effector 20 in response to determining that the surgical tool 22 is interacting with a virtual constraint.

The virtual constraints may be a temporary virtual constraint. A temporary virtual constraint may be defined as a virtual constraint that may be removed/deactivated by the controller 30. For example, in some instances, the controller 30 may remove/deactivate a temporary virtual constraint after a predetermined amount of time has passed. As another example, the controller 30 may remove/deactivate a temporary virtual constraint in response to completion of a step of a surgical procedure. As another example, the controller 30 may remove/deactivate a temporary virtual constraint in response to an action by the operator of the surgical system 10. For instance, the controller 30 may remove a temporary virtual constraint in response to the operator interacting with the input/output devices 40, 42, 43.

As will be explained in greater detail below, the boundary generator 66 may be configured to generate virtual constraints during any of the methods described herein. For example, the boundary generator 66 may be configured to generate virtual constraints during the method 100. Additionally, the virtual constraints may be generated prior to the method 100.

In some implementations, the virtual constraints may be generated offline rather than on the manipulator computer 26 or navigation computer 36. Thereafter, the virtual constraints may be utilized at runtime by the manipulator controller 60.

### b. Tool Path Generator

As shown in FIG. 2, the controller 30 includes a tool path generator 68. The tool path generator 68 is a software module that may be implemented on the manipulator controller 60. Alternatively, the tool path generator 68 may be implemented on any other suitable component of the system 10.

The tool path generator 68 generates a path for the manipulator 14 and/or the end effector 20 to traverse, such as for removing sections of the anatomy A to receive an implant. One exemplary system and method for generating the tool path 70 is explained in U.S. Patent No. 9,937,014, entitled, "System and Method of Controlling a Surgical Tool During Autonomous Movement of the Surgical Tool," the disclosure of which is hereby incorporated by reference.

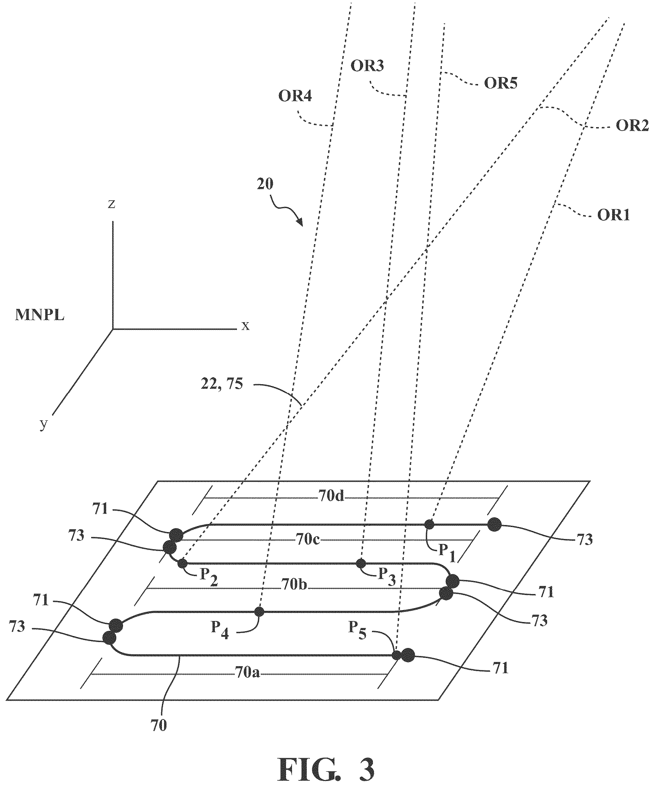

The tool path generator 68 generates a tool path 70 relative to the surgical site and the controller 30 controls the manipulator 14 to facilitate movement of the end effector 20 and surgical tool 22 along the tool path 70 for treating the surgical site. One example path 70 is shown in FIG. 3. The tool path generator 68 determines the tool path 70 for the surgical tool 22 of the end effector 20 to move along in the manipulator coordinate system MNPL. In addition, the tool path generator 68 generates path data related to the tool path 70 and communicates the path data to the manipulator controller 60. The tool path generator 68 may provide pre-operative, and/or intra-operative path data to the manipulator controller 60 such that the tool path 70 may be updated at any time during the surgical procedure.

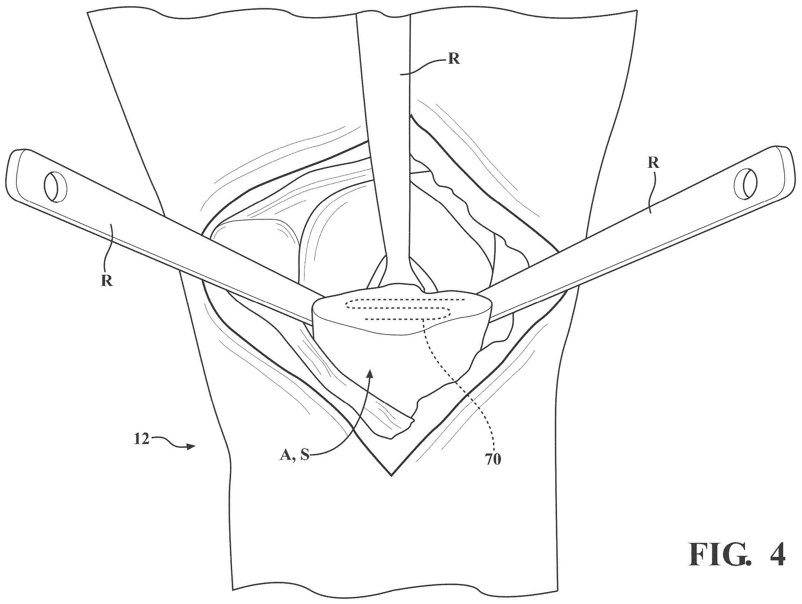

The tool path 70 may be defined in, or through, tissue of the patient 12. For example, the tool path 70 may be defined to allow the surgical tool 22 to cut and remove a volume of bone such that the bone may receive an implant. The tool path 70 may correspond to a particular volume of bone to be cut to receive a specific geometry (e.g., size and shape) of implant. In the instance of FIG. 4, the surgical site S is the tibia of a patient 12 and the tool path 70 is shown as being defined on a surface of the tibia. In other instances, the tool path 70 may be defined in any other tissue of the patient, such as any bone or soft tissue of the patient 12.

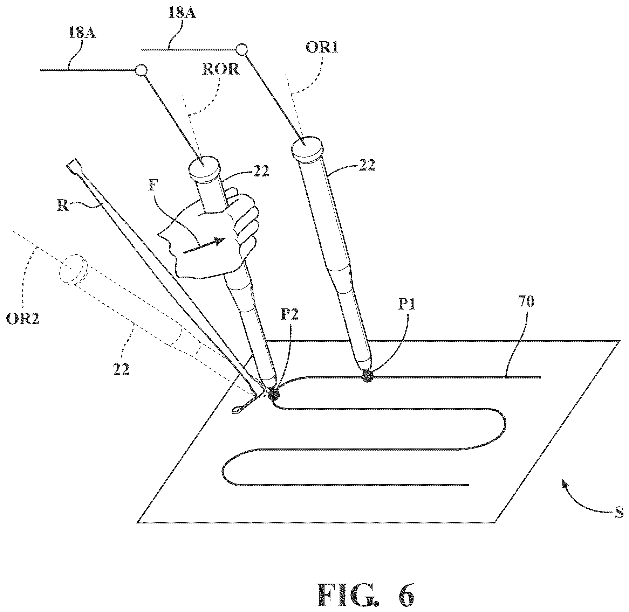

The tool path 70 may be divided into one or more segments. For example, in the instance of FIG. 3, the tool path 70 is divided into four segments 70a-70d, with each segment 70a-70d having a first endpoint 71 and a second endpoint 73. For each segment 70a-70d of the tool path 70, the tool path generator 68 may process the data provided by the controller 30 to generate path segment data. Although only four segments 70a-70d are described and illustrated in FIG. 3, those skilled in the art appreciate that the tool path 70 may be divided into any suitable number of segments. For instance, referring to FIG. 6, the tool path 70 shown could comprise seven segments with each straight portion being separate segments and each curved portion being separate segments. The segments may each be of similar length or may be of varying lengths.

The tool path generator 68 may generate a plurality of tool path points P along the tool path 70. The plurality of tool path points P may be any point along the tool path 70, with each tool path point P corresponding to a target position of the surgical tool 22. In the instance of FIG. 3, the plurality of tool path points P may include the endpoints 71,73. Additionally, the plurality of tool path points P may include points located along each segment 70a-70d, such as point P1-P5. The tool path 70 may include any suitable number of tool path points P. In some instances, the controller 30 may include a tool path interpolator configured to determine target positions of the surgical tool 22 along the tool path 70 and corresponding tool path points P. Example of the tool path interpolators can be like those described in U.S. Patent Application No. 18/082,996, entitled, "Robotic Systems, Methods and Software Programs For Modifying Tool Operation Based on Tissue Parameters," and U.S. Patent No. 10,117,713, entitled "Robotic Systems and Methods for Controlling a Tool Removing Material from a Workpiece", the disclosures of which is hereby incorporated by reference.

The tool path generator 68 may generate the path data using any suitable input. For example, the tool path generator 68 may generate path data using data provided by the controller 30 and relating to the patient 12, the surgical tool 22, the implant, and/or any other object located at the surgical site S. The data input into the tool path generator 68 may include femur and/or tibia pose data, surgical tool 22 pose data, other object pose data, imaging data (e.g., CT/MRI data), data defining the shape of a boundary across which the surgical tool 22 is not to extend and/or data defining the volume of tissue to be removed by the surgical tool 22, implant data, and data relating to the surgeon's setting of the location of the boundary. As another example, the tool path generator 68 may generate the path data based on a type of procedure and/or a preference for the surgical procedure. For instance, the tool path generator 68 may generate the path data to optimize cutting efficiency of the surgical tool 22 along the tool path 70, cutting speed of the surgical tool 22 along the tool path 70, and/or to reduce a distance traveled by the surgical tool 22 while moving along the tool path 70.

Additionally, the tool path 70 may be registered to a tracked anatomy A of the patient 12. For example, referring to FIG. 4, the tool path 70 may be registered to the tibia of the patient 12. In such an instance, a location of the tool path 70 is fixed relative to the tibia during movement of the patient 12.

In some implementations, the tool path 70 may be generated offline rather than on the manipulator computer 26 or navigation computer 36. Thereafter, the tool path 70 may be utilized at runtime by the manipulator controller 60.

### c. Modes of Operation of the Controller

The controller 30 may use a variety of operation modes to control the manipulator 14. For example, the controller 30 may control the manipulator 14/robotic arm 18A to interact with the surgical site S using semi-autonomous, automatic, manual, and guided-manual modes of operation.

In the semi-autonomous and automatic modes, the controller 30 directs movement of the robotic arm 18A and/or the end effector 20 at the surgical site S. In such instances, the controller 30 obtains a tool path 70 from the tool path generator 68 and controls the manipulator 14 to facilitate movement of the tool along the tool path 70. More particularly, in the semi-autonomous and automatic modes, the controller 30 may model the robotic arm 18A and/or the end effector 20 as a virtual rigid body and determine forces and torques to apply to the virtual rigid body to advance and constrain the robotic arm 18A and/or the end effector 20 along a tool path 70. Movement of the tool 22 in the semi-autonomous and automatic modes is constrained in relation to the virtual constraints generated by the boundary generator 66 and/or the path generator 68.

In the semi-autonomous mode, the controller 30 is capable of moving the robotic arm 18A and/or end effector 20 free of operator assistance. Free of operator assistance may mean that an operator does not physically move the robotic arm 18A and/or end effector 20 by applying external forces/torques to move the robotic arm 18A and/or end effector 20. Instead, the operator may use some form of control to manage starting and stopping of movement. For example, the operator may hold down a button of a control to start movement of the robotic arm 18A and/or end effector 20 and release the button to stop movement of the robotic arm 18A and/or end effector 20. Alternatively, the operator may press a button to start movement of the robotic arm 18A and/or end effector 20 and press a button to stop motorized movement of the robotic arm 18A and/or end effector 20 along the tool path 70 generated by the tool path generator 68. The controller 30 uses motorized movement to advance the robotic arm 18A and/or end effector 20 in accordance with pre-planned parameters. An example of the semi-autonomous mode is described in U.S. Patent No. 9,119,655, entitled, "Surgical Manipulator Capable of Controlling a Surgical Instrument in Multiple Modes," the disclosure of which is hereby incorporated by reference.

In the manual mode and guided-manual mode, the controller 30 is configured to control movement of the robotic arm 18A and/or end effector 20 based on external forces/torques applied to a component of the manipulator 14, such as the robotic arm 18A and/or the end effector 20. The external forces/torques may also be applied to a haptic device, such as the haptic device described in U.S. Patent No. 10,350,012, entitled, "Method and Apparatus for Controlling a Haptic Device," the disclosure of which is hereby incorporated by reference.

In the manual mode, the robotic arm 18A is freely moveable and the operator may manually direct, and the controller 30 control, movement of the robotic arm 18A and/or end effector 20 at the surgical site S. For instance, the operator may physically contact and apply external forces/torques to a component of the manipulator 14 to direct movement of the robotic arm 18A and/or end effector 20. Movement of the robotic arm 18A and/or end effector 20 in the manual mode may also be constrained in relation to the virtual constraints generated by the boundary generator 66 and/or path generator 68.

In the guided-manual mode, the system 10 guides movement of the end effector 20 along the tool path 70 generated by the tool path generator 68, in response to external forces/torques applied to a component of the manipulator 14. In such instances, the controller 30 obtains a tool path 70 from the tool path generator 68 and controls the manipulator 14 to facilitate movement of the tool along the tool path 70 in response to external forces/torques. For instance, the operator may physically contact and apply external forces/torques to a component of the manipulator 14 to guide movement of the end effector 20 along the tool path 70. For example, the operator may grasp and apply external forces/torques to the end effector 20 and/or surgical tool 22 to move the end effector 20 along the tool path 70. The guided-manual mode relies on external forces/torques applied to the manipulator 14 to advance the tool 22, but such advancement, instead of merely emulating the movement that would have occurred based on the external forces/torques applied, is actively controlled to be along the tool path 70. As such, during guided-manual mode, a component of the end effector 20, such as a center point of the surgical tool 22, is constrained along the tool path 70. The guided-manual mode is further described in U.S. Patent No. 11,564,761, entitled, "Systems and Methods for Controlling Movement of a Surgical Tool Along a Predefined Path" the disclosure of which is hereby incorporated by reference.

### d. Orientation Generator

As shown in FIG. 2, the controller 30 includes an orientation generator 74. The orientation generator 74 is a software module that may be implemented on the manipulator controller 60. Alternatively, the orientation generator 74 may be implemented on any other suitable component of the system 10.

The orientation generator 74 can generate preferred orientations of the end effector 20 and surgical tool 22 with respect to the predetermined tool path 70. One exemplary system and method for generating the preferred orientations is explained in U.S. Patent No. 9,937,014, entitled, "System and Method of Controlling a Surgical Tool During Autonomous Movement of the Surgical Tool," the disclosure of which is hereby incorporated by reference, wherein the preferred orientations are referred to as acceptable orientations.

The orientation generator 74 may generate a preferred orientation of the surgical tool 22 for each tool path point P generated by the tool path generator 68. For example, in the instance of FIG. 3, the orientation generator 74 generates preferred orientations OR1-OR5 corresponding to each of the tool path points P1-P5, respectively. Additionally, or alternatively, the orientation generator 74 may also generate a preferred range of orientations of the surgical tool 22 for each tool path point P.

Referring to FIG. 3, the controller 30 may autonomously move the surgical tool 22 along the tool path 70 in the orientations OR1-OR5 while the controller 30 controls the manipulator 14 in the semi-autonomous and automatic modes. The manipulator controller 60 may communicate with the path generator 68 and the orientation generator 74 to instruct the manipulator 14 to autonomously move the surgical tool 22 along the tool path 70 and in the preferred orientations OR1-OR5. In the semi-autonomous mode, the controller 30 may autonomously move the surgical tool 22 along the tool path 70 in the preferred orientations OR1-OR5 without the operator handling the surgical tool 22 or free of operator assistance. Additionally, in the guided-manual mode, the controller 30 may constrain the surgical tool 22 such that the surgical tool 22 moves along the tool path 70 in the orientations OR1-OR5 in response to external forces/torques applied to a component of the manipulator 14.

The orientation generator 74 may generate the preferred orientations based on any suitable input. The orientation generator 74 may receive, as an input, femur and/or tibia pose data, other object pose data, imaging data (e.g., CT/MRI data), data defining a virtual constraint across which the surgical tool 22 is not to extend and/or data defining a volume of tissue to be removed by the surgical tool 22, implant data, data relating to the surgeon's setting of the location of the boundary, data relating to a preference for the surgical procedure (e.g. optimizing cutting efficiency and/or optimizing cutting speed), data defining a location of the localizer 44, data defining the tool path 70 along which the surgical tool 22 is to traverse, and/or a type of surgical procedure.

The orientation generator 74 may generate the preferred orientations using any suitable method. In some instances, the orientation generator 74 may generate the preferred orientations to optimize cutting efficiency of the surgical tool 22 along the tool path 70. For instance, if the surgical procedure is a bone milling procedure, the surgical tool 22 may have a preferred orientation or preferred range of orientations to avoid cutting inefficiencies. The surgical tool 22 may more efficiently cut at certain orientations as compared with others. Parameters regarding cutting efficiency of the surgical tool 22 in various scenarios may be stored in memory and accessed by the orientation generator 74. Cutting efficiency of the surgical tool 22 may be determined according to any suitable method. In one exemplary implementation when cutting the femur F, the orientation generator 74 selects the orientation of the surgical tool 22 that results in the orientation of the surgical tool 22 being parallel to a longitudinal axis of the femur F. In this implementation, the orientation generator 74 receives data defining the tool path 70 and femur pose data including the orientation of the longitudinal axis.

The manipulator controller 60 may communicate with the orientation generator 74 to determine the preferred orientations of the surgical tool 22 with respect to the tool path 70. The orientation generator 74 generates orientation data relating to the preferred orientations. For any given moment or tool path point P along the tool path 70, the preferred orientation may be a single preferred orientation or a plurality of preferred orientations. The orientation generator 74 may then communicate the orientation data relating to the preferred orientations of the surgical tool 22 to the manipulator controller 60. The manipulator controller 60 processes the orientation data and directs the manipulator 14 to orient the surgical tool 22 when the surgical tool 22 moves autonomously along the tool path 70, as disclosed in U.S. Patent No. 9,119,655, entitled, "Surgical Manipulator Capable of Controlling a Surgical Instrument in Multiple Modes," the disclosure of which is hereby incorporated by reference.

### e. Input/Output Devices

The input/output devices 40, 42, 43 may be coupled to the controller 30 and the controller 30 may detect an input from the input/output devices 40, 42, 43. For example, the detected input may be a detected actuation of the footswitch 43 or a detected interaction with an interactive touchscreen display 40, 42. The detected input may also be a gesture, gaze, voice, and/or user head motion detected by a head-mounted device. In this way, the operator may interact with the input/output devices 40, 42, 43 to communicate with the software modules shown in FIG. 2. For example, the input/output devices 40, 42, 43 may be actuated by an operator to input information into and/or select/control certain aspects of the manipulator controller 60, the manipulator computer 26, the navigation controller 62, and/or the navigation computer 36. The input/output devices 40, 42, 43 may communicate with the controller 30 via a user interface software. The user interface software may run on the manipulator computer 26 and navigation computer 36, or on a separate device from the manipulator computer 26 and navigation computer 36.

The controller 30 may provide haptic, audible, and/or visual feedback to an operator of the surgical system 10. The controller 30 may provide haptic, audible, and/or visual feedback to an operator to provide the operator with guidance based on virtual constraints and/or to provide a notification/indication to the operator. The controller 30 may implement haptic, audible, and/or visual feedback to any suitable component of the surgical system. For example, the controller 30 may implement haptic feedback to one or more of the input/output devices 40, 42, 43, to the end effector 20, to the robotic arm 18A, and/or to a hand-held robotic device, such as a hand-held robotic saw, drill, or tool guide. As another example, the controller 30 may implement haptic feedback via a haptic device, such as the haptic device described in U.S. Patent No. 10,350,012, entitled, "Method and Apparatus for Controlling a Haptic Device," the disclosure of which is hereby incorporated by reference. The controller 30 may implement audible feedback via a speaker of the surgical system 10, such as a speaker of the input/output devices 40, 42, 43. The controller 30 may implement visual feedback via a display of the surgical system 10, such as the display 38 of the input/output devices 40, 42 and/or a display of a head-mounted device.

Furthermore, the haptic, audible, and/or visual feedback may be any suitable feedback means. For example, the haptic feedback may be a vibration pattern or a button-clicking sensation provided to a component of the surgical system, such as one or more of the input/output devices 40, 42, 43. The audible feedback may be any sound provided by a component of the surgical system, such as one or more of the input/output devices 40, 42, 43. For instance, the sound may be a button-clicking sound. The visual feedback may be any notification and/or 2D/3D image provided by a component of the surgical system, such as one or more of the input/output devices 40, 42, 43. For instance, the notification and/or 2D/3D image may be provided by a display of the surgical system 10, such as the display 38 of the interactive touchscreen devices 40, 42 and/or a display of a head-mounted device.

### III. REORIENTATION OF THE SURGICAL TOOL

### a. Overview

The controller 30 may determine whether the surgical tool 22 has been reoriented. Reorientation may be defined as an alteration of an orientation of the surgical tool 22. In some instances, the operator may apply an external force to a component of the manipulator 14 to reorient the surgical tool 22. In some instances, reorientation of the surgical tool 22 may occur even when the operator does not apply an external force. For example, forces may be inadvertently applied to the surgical tool 22 causing a reorientation of the surgical tool 22. In one such instance, the surgical tool 22 may collide with an obstacle and the obstacle may apply an external force to the surgical tool 22, reorienting the surgical tool 22.

Reorientation of the surgical tool 22 may prevent a collision between the surgical tool 22 and an obstacle located within the surgical site S. The obstacle may be any obstacle inhibiting the surgical procedure. For example, the obstacle may be a retractor located within the surgical site S, another surgical tool 22 located within the surgical site S, an arm of a surgical assistant, or a tissue of the patient 12. For instance, referring to FIG. 4, the surgical tool 22 may collide with a retractor R during movement along the predetermined tool path 70 in the automatic, semi-autonomous, or guided-manual mode. In such instances, the surgical tool 22 may be reoriented to prevent a collision between the surgical tool 22 and the retractor R. Such obstacles may have been undetected by the navigation system 32 and therefore, unaccounted for during generation of the predetermined tool path 70. For example, an obstacle and/or a tracker 52, 54, 56 may not have been located in the line of sight of the localizer 44 and the boundary generator 66 may not have generated a virtual constraint corresponding to the obstacle and/or the object coupled to the tracker 52, 54, 56. As another example, a position of such obstacles may have altered after generation of the tool path 70 and/or during movement of the end effector 20 along the tool path 70.

Reorientation of the surgical tool 22 may also be based on the mobility/ergonomics of the operator and/or a preference of the operator. In such instances, the surgical tool 22 may be reoriented to allow the surgical tool 22 to be moved in accordance with the desires and/or ability of the operator. For example, in the guided-manual mode, the controller 30 may constrain the surgical tool 22 such that the surgical tool 22 moves along the tool path 70 in preferred orientations in response to external forces/torques applied to a component of the manipulator 14. However, in some instances, such preferred orientations may not be ergonomically friendly as they may put unnecessary stress on the operator. In such instances, the surgical tool 22 may be reoriented to provide a more ergonomically friendly orientation for the operator. As another example, the operator may have a preferred wrist positioning and/or hand preference (e.g., left hand or right hand) for grasping and applying external forces/torques to the end effector 20. In such instances, the surgical tool 22 may be reoriented to allow the operator to grasp the end effector 20 and/or surgical tool 22 using the preferred wrist position and/or hand preference.

Reorientation of the surgical tool 22 may be caused by limitations of the manipulator. For example, the controller 30 may be prevented from moving along the tool path 70 in a preferred orientation due to one or more of a workspace boundary of the manipulator 14, a range of motion of the manipulator 14, a limit of a joint (J) of the manipulator 14, and a singularity of the manipulator 14. For instance, the range of motion of the manipulator 14 may be limited at a point along the tool path 70, preventing the surgical tool 22 from achieving a preferred orientation at the point. In such instances, the surgical tool 22 may be reoriented to allow the manipulator 14 to continue movement along the tool path 70, despite a limitation in the range of motion of the manipulator 14 at the point along the tool path 70.

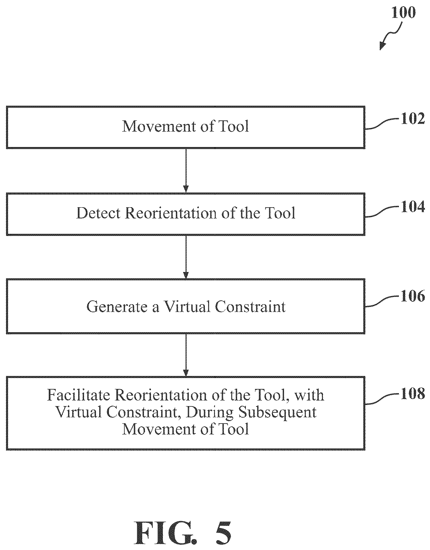

Referring to FIG. 5, a method 100 is provided of facilitating reorientation of a surgical tool 22. The method 100 includes a step 102 of controlling the manipulator 14 to facilitate movement of the surgical tool 22 for treating the surgical site S; a step 104 of detecting an initial reorientation of the surgical tool 22 during movement of the surgical tool 22; a step 106 of generating a virtual constraint; and a step 108 of facilitating subsequent reorientation of the surgical tool 22 with the generated virtual constraint during subsequent movement of the surgical tool 22. Herein, "initial reorientation" is used to describe reorientations that are detected during step 104 but "initial" does not necessarily mean the first reorientation. The initial reorientation may occur after other reorientations. Additionally, "subsequent reorientation" is used herein to describe a reorientation that occurs due to the virtual constraint interacting with the surgical tool 22 during step 108.

During step 102, the controller 30 may control the manipulator 14 to facilitate movement of the surgical tool 22 for treating the surgical site S. The controller 30 may facilitate movement of the surgical tool 22 in accordance with any of the above-described modes of operation of the controller 30, such as automatic, semi-autonomous, manual, or guided-manual mode. In instances where the controller 30 controls the manipulator 14 in the automatic, semi-autonomous, or guided-manual modes, the controller 30 obtains a tool path 70 from the tool path generator 68 and controls the manipulator 14 to facilitate movement of the tool along the tool path 70 for treating the surgical site S.

During step 104, the controller 30 detects the initial reorientation of the surgical tool 22. The initial reorientation may be a manual reorientation responsive to external forces/torques applied to the end effector 20 and/or surgical tool 22 by an operator/user. In such instances, the operator may apply an external force to the end effector 20 and/or surgical tool 22 causing the initial reorientation of the surgical tool 22. The initial reorientation may also be caused by a collision with an obstacle. In such instance, the obstacle may apply an external force to the end effector 20 and/or surgical tool 22 causing the initial reorientation of the surgical tool 22.

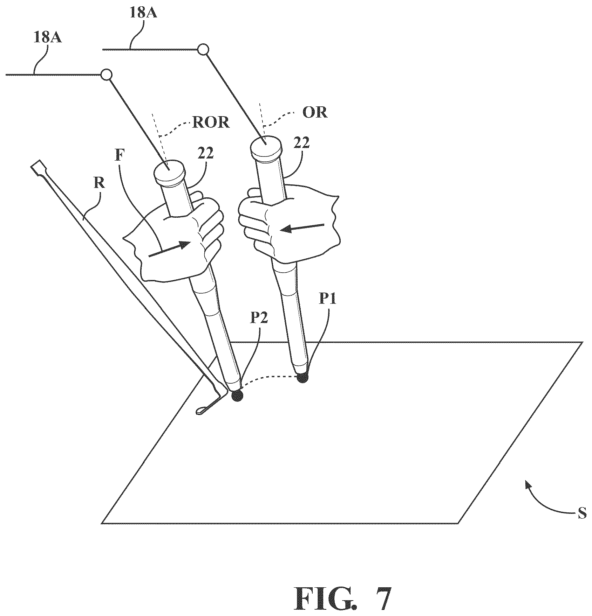

Example initial reorientations of the surgical tool 22 are shown in FIGS. 6 and 7. As shown, the surgical tool 22 is coupled to the robotic arm 18A of the manipulator 14 and an obstacle (shown as the retractor R) is located in the path of the surgical tool 22. In FIG. 6, the surgical tool 22 travels along and is constrained to a tool path 70 in any one of the automatic, semi-autonomous, or guided manual mode. The tool path 70 of FIG. 6 includes tool path points P1, P2 and corresponding predetermined orientations OR1, OR2. In the instance of FIG. 6, the initial reorientation occurs at point P2 on the tool path 70, where an external force F is applied to the surgical tool 22. The external force F reorients the surgical tool 22 to orientation ROR, preventing the surgical tool 22 from being oriented at orientation OR2, and preventing the surgical tool 22 from colliding with the retractor R. In FIG. 7, the manipulator 14 controls movement of the surgical tool 22 in the manual mode. In other words, the surgical tool 22 does not travel along a tool path 70. Instead, the manipulator 14 controls movement of the surgical tool 22 based on an external force provided by an operator. As shown, the surgical tool 22 moves from a point P1 to a point P2. The initial reorientation occurs at point P2, where an external force F is applied to the surgical tool 22, moving the surgical tool 22 from point P1 to P2, reorienting the surgical tool 22 from orientation OR to orientation ROR, and preventing the surgical tool 22 from colliding with the retractor R.

In some instances, during step 104, the controller 30 may also record or detect a location at which the initial reorientation occurred. As will be explained in greater detail below, the controller 30 may record or detect the location at which the initial reorientation occurred by detecting the location relative to the tool path 70, the surgical site S, and/or an obstacle to be avoided.

During step 104, the controller 30 may detect the initial reorientation during any above-described mode of operation of the controller 30. For instance, the controller 30 may detect the initial reorientation during movement of the surgical tool 22 along a predetermined tool path 70 while the controller 30 controls the manipulator 14 in the automatic, semi-autonomous, or guided- mode. Alternatively, the controller 30 may detect the initial reorientation during movement of the surgical tool 22 while the controller controls the manipulator 14 in the manual mode.

During step 106, the controller 30 generates virtual constraint(s) in response to detecting the initial reorientation of the surgical tool 22 during step 104. Specifically, the virtual constraints may be generated by the boundary generator 66. The virtual constraints generated by the boundary generator 66 may be any suitable type of virtual constraint, such as a virtual mesh, a particle guide, and/or a stereotactic interaction feature.

As such, the initial reorientation during step 104 of the surgical tool 22 may cause the controller 30 to generate a virtual constraint during step 106. Advantageously, in instances where an obstacle was undetected by the navigation system 32, the surgical tool 22 may be reoriented to avoid the undetected obstacle, the reorientation may be detected during step 104, and the controller 30 may generate a virtual constraint corresponding to the undetected obstacle during step 106. For example, in instances where the surgical tool 22 moves along the predetermined tool path 70, the surgical tool 22 may be reoriented to avoid an obstacle and the controller 30 may generate a virtual constraint corresponding to the obstacle. Similarly, in instances where the surgical tool 22 is moved in the manual mode, the surgical tool 22 may be reoriented to avoid an obstacle and the controller 30 may generate a virtual constraint corresponding to the obstacle.

During step 108, the virtual constraint facilitates reorientation of the surgical tool 22 in response to interaction between the surgical tool 22 and the virtual constraint during a subsequent (e.g., later or future) movement of the surgical tool 22. In some instances, the virtual constraint generated during step 106 may be generated at a location of the recorded initial reorientation. During subsequent movement of the surgical tool 22, the surgical tool 22 may again be located at the recorded location of the initial reorientation or near the recorded location of the initial reorientation. In such instances, the surgical tool 22 may interact with the virtual constraint generated during step 106 and the generated virtual constraint may facilitate the subsequent reorientation of the surgical tool 22. For example, in instances where the controller 30 controls the manipulator 14 in the automatic, semi-autonomous, or guided-manual mode, the controller 30 may control the surgical tool 22 to continue movement along the same tool path 70, to restart movement along the same tool path 70, or move along a different tool path during subsequent movement of the surgical tool 22. Such movement may cause the surgical tool 22 to be again located at the location of the detected initial reorientation or near the location of the detected initial reorientation. In instances where the controller 30 controls the manipulator 14 in the manual mode, the controller 30 may control the surgical tool 22 based on external forces/torques provided by an operator, which may cause the surgical tool 22 to again be located at the location of the detected initial reorientation or near the location of the detected initial reorientation.

Steps 102-108 of the method 100 of facilitating reorientation may be repeated any suitable number of times. For example, an instance of the step 104 of detecting the initial reorientation of the surgical tool 22 may occur each time external forces/torques are applied to the surgical tool 22. Similarly, an instance of the step 106 of generating a virtual constraint may occur each time an initial reorientation of the surgical tool 22 is detected during an instance of step 104; and an instance of the step 108 of facilitating reorientation of the surgical tool 22 may occur each time a virtual constraint generated during an instance of step 106 interacts with the surgical tool 22.

In one such implementation, an operator may apply a first external force to the surgical tool 22, and the controller 30 may detect a first initial reorientation during a first instance of step 104. The controller 30 may then generate a first virtual constraint during a first instance of step 106. As follows, during a first instance of step 108, the first virtual constraint may interact with the surgical tool 22 during subsequent movement of the surgical tool 22 to facilitate a first subsequent reorientation of the surgical tool 22. Additionally, an operator may apply a second external force to the surgical tool 22, and the controller 30 may detect a second initial reorientation during a second instance of step 104. The controller 30 may then generate a second virtual constraint during a second instance of step 106. As follows, during a second instance of step 108, the second virtual constraint may interact with the surgical tool 22 during subsequent movement of the surgical tool 22 to facilitate a second subsequent reorientation of the surgical tool 22. Each of the first and second initial reorientations may occur during movement of the surgical tool 22 along a tool path 70, or during movement of the surgical tool 22 in the manual mode.

### b. Control Schemes of the Controller

As previously stated, external forces/torques may be applied to the end effector 20 and/or surgical tool 22 to cause the initial reorientation of the surgical tool 22. The controller 30 may control the manipulator 14/robotic arm 18A based on the external forces/torques to implement the initial reorientation. Furthermore, the controller 30 may control the manipulator 14/robotic arm 18A to implement the initial reorientation using any suitable control scheme, such as an impedance control scheme or an admittance control scheme.

In instances where the controller 30 controls the manipulator 14 based on an impedance control scheme, the controller 30 allows the external forces/torques applied to the end effector 20 and/or surgical tool 22 to alter a pose of the manipulator 14. In one such implementation, the joints (J) of the robotic arm 18A may be passively driven such that the external forces/torques cause displacement of the robotic arm 18A and alter a pose of the manipulator 14. In some instances, the altered pose may be a pose where the manipulator 14 interacts with a virtual constraint. As such, the controller 30 may determine the altered pose of the manipulator 14 to determine whether the altered pose of the manipulator 14 causes an interaction with a virtual constraint. For example, the controller 30 may determine the altered pose of the manipulator 14 by determining positions of the joints (J) of the robotic arm 18A. The controller 30 may then command reactive forces/torques based on the altered pose of the manipulator 14. The commanded reactive forces/torques may include a constraint force applied by the virtual constraint for constraining the surgical tool 22. The commanded reactive forces/torques may be applied to joints (J) of the robotic arm 18A.

In instances where the controller 30 controls the manipulator 14 based on an admittance control scheme, the controller 30 may detect the external forces/torques applied to the end effector 20 and/or surgical tool 22 and control movement of the manipulator 14 based on the detected forces/torques. In one such implementation, the manipulator 14 may include a force-torque sensor, which may detect the external forces/torques. In another such implementation, the manipulator 14 may include a current sensor, which may detect a current provided to a motor of the manipulator 14 to detect the external forces/torques. Upon detecting the external forces/torques, the controller 30 may command a pose of the manipulator 14 (e.g., the controller 30 may command a pose of the joints (J) of the robotic arm 18A) based on the detected external forces/torques. Additionally, the controller 30 may command the pose of the manipulator 14 based constraint forces applied by a virtual constraint. For example, in some instances, the external forces/torques would displace the manipulator 14 to a pose where the manipulator 14 would interact with a virtual constraint. In such instances, the controller 30 may determine the commanded pose based on the external forces/torques and based on a constraint force applied by the virtual constraint.

Impedance and admittance control schemes are further described in U.S. Patent No. 10,350,012, entitled, "Method and Apparatus for Controlling a Haptic Device" and U.S. Patent No. 10,327,849, entitled, "Robotic System and Method For Backdriving The Same", the disclosures of which are hereby incorporated by reference.

### c. Detecting Reorientation

The controller 30 may detect the initial reorientation during step 104 by detecting a parameter of the end effector 20 after occurrence of the initial reorientation. The parameter may be any suitable parameter for detecting the occurrence of the initial reorientation. For example, the parameter may be one or more of a direction of external forces/torques applied to the surgical tool 22, a magnitude of external forces/torques applied to the surgical tool 22, a displacement of the surgical tool 22, a velocity of the surgical tool 22, and an acceleration of the surgical tool 22. Referring to FIGS. 6 and 7, an operator applies an external force F to the surgical tool 22 at point P2 to cause the initial reorientation. The controller 30 may detect the initial reorientation by detecting a direction of the external force F, a magnitude of the external force F, a displacement of the surgical tool 22 caused by the external force F, a velocity of the surgical tool 22 during the displacement, and/or an acceleration of the surgical tool 22 during the displacement.

The controller 30 may detect the initial reorientation during step 104 by determining whether the detected parameter of the surgical tool 22 is greater than a threshold value. For example, the controller 30 may determine whether a direction of the external force F of FIGS. 6 and 7 is greater than a threshold angle value. In other instances, the controller 30 may additionally, or alternatively, determine whether a magnitude of the external force F is greater than a threshold magnitude value. Similarly, the controller 30 additionally, or alternatively, determine whether the displacement of the surgical tool 22 is greater than a threshold displacement value, whether the velocity of the surgical tool 22 is greater than a threshold velocity value, and/or whether the acceleration of the surgical tool 22 is greater than a threshold acceleration value.

The above-described threshold values may be determined by the controller 30. In some instances, the controller 30 may determine the above-threshold values based on a pose of the surgical tool 22 and the anatomy A of the patient 12, both of which may be tracked by the navigation system 32. For example, the controller 30 may estimate a threshold magnitude value for the external force F for resecting the anatomy A at the tracked position of the surgical tool 22. Should the external force F be greater than the threshold magnitude value, the controller may determine the external force F is greater than necessary for resecting the anatomy A at the tracked position and that the initial reorientation has occurred. In this way, the controller 30 may also estimate a threshold angle value, a threshold displacement value, threshold velocity value, and/or threshold acceleration value for the surgical tool 22.

The controller 30 may detect the initial reorientation during step 104 by determining whether the surgical tool 22 has deviated from the tool path 70. In such instances, an obstacle may prevent the surgical tool 22 from moving along the tool path 70. For example, a retractor R may be located directly on the tool path 70, blocking the surgical tool 22 from traveling along the tool path 70. In such an instance, the surgical tool 22 may be reoriented in a manner that displaces the surgical tool 22 from the tool path 70. The controller 30 may be configured to detect such an initial reorientation by recording a location of the surgical tool 22 relative to the tool path 70. In some instances, the controller 30 may detect the initial reorientation by determining whether an amount of displacement of the surgical tool 22 from the tool path 70 is greater than a threshold displacement value.

The controller 30 may detect the initial reorientation during operation based on the impedance control scheme. In such instances, external forces/torques applied to the manipulator 14 may cause the surgical tool 22 to move to a current pose. The controller 30 may then detect the initial reorientation based on the current pose. In some instances, the controller 30 may detect the initial reorientation by comparing the detected current pose with one or more previous poses of the surgical tool 22. For example, the controller 30 may determine a displacement of the surgical tool 22 based on the current pose and a previous pose of the surgical tool 22.

The controller 30 may detect the initial reorientation during operation based on the admittance control scheme. In such instances, the controller 30 may detect the external forces/torques applied to the manipulator 14 and determine a commanded pose of the surgical tool 22. The controller 30 may then cause movement of the surgical tool 22 from a current pose to the commanded pose. In such instances, the controller 30 may detect the initial reorientation based on the commanded pose. In some instances, the controller 30 may detect the initial reorientation by comparing the commanded pose with the current pose and/or one or more previous poses of the manipulator 14. For example, the controller 30 may determine a displacement of the surgical tool 22 based on the commanded pose and the current pose of the manipulator 14. In some instances, the controller 30 may detect a parameter of the surgical tool 22. For example, the controller 30 may sense a velocity of the surgical tool 22 while the surgical tool 22 is moved to the commanded pose.

In some implementations, the surgical system 10 may include a user interface configured to receive an input, and the controller 30 may detect a reorientation of the surgical tool 22 based on the user interface receiving an input. The user interface may be any of the previously described input/output devices 40, 42, 43.

In an example implementation, the controller 30 may determine that a magnitude of a force applied to the surgical tool 22 exceeds the threshold force value and provide an indication of a potential reorientation to the operator via one or more of the interactive touchscreen displays 40, 42. In some instances, the controller 30 may notify an operator of a reorientation and request confirmation from the operator via a display of the surgical system 10, such as the display 38 of one or more of the interactive touchscreen displays 40, 42 and/or a display of a head-mounted device before determining that a reorientation has been detected. In instances where the input/output devices 40, 42, 43 include a head-mounted device, the head-mounted device may be configured to receive the confirmation by tracking a gesture, a gaze, and/or a head motion of the operator. The head-mounted device may also include a microphone and/or audio sensor configured to receive voice instructions/commands as the confirmation from the user.

In another example implementation, a button may be located on the surgical tool 22, and the button may be depressed when the operator wants to manually reorient the surgical tool 22. For example, the button may be located on the end effector 20 and/or the surgical tool 22. In some instances, the button may be the footswitch 43. The button may also be located on a hand-held pendant device, as shown and described in U.S. Patent No. 9,119,655, entitled, "Surgical Manipulator Capable of Controlling a Surgical Instrument in Multiple Modes," the disclosure of which is hereby incorporated by reference. Depressing the button signals that the operator desires to reorient the surgical tool 22 and the controller 30 may detect a reorientation of the surgical tool 22 based on depression of the button.

The controller 30 may notify an operator of a detection of the initial reorientation. The controller 30 may provide haptic, audible, and/or visual feedback to an operator of the surgical system 10 in response to detecting the initial reorientation. For example, the controller 30 may provide a vibration pattern to one or more of the input/output devices 40, 42, 43 in response to detecting the initial reorientation. As another example, the controller 30 may provide a visual notification via a display of surgical system 10 in response to detecting the initial reorientation. The display may be the display 38 of the interactive touchscreen devices 40, 42 and/or a display of a head-mounted device. In some instances, the controller 30 may request confirmation of the initial reorientation from the operator in response to detection of the initial reorientation. The controller 30 may request confirmation via a display of the surgical system 10. In instances where the input/output devices 40, 42, 43 include a head-mounted device, the head-mounted device may be configured to receive the confirmation by tracking a gesture, a gaze, and/or a head motion of the operator. The head-mounted device may also include a microphone and/or audio sensor configured to receive voice instructions/commands as the confirmation from the user.

### d. Generating Virtual Constraint

The virtual constraints generated by the controller 30 during step 106 may be any suitable type of virtual constraint, such as a virtual mesh, a particle guide, and/or a stereotactic interaction feature.

The virtual constraint generated by the boundary generator 66 may be generated relative to a recorded location of any reorientation of the surgical tool 22. In such instances, the controller 30 may record a location of the reorientation of the surgical tool 22 during step 104 and generate a virtual constraint relative to the location during step 106. For example, referring to FIGS. 6 and 7, the controller 30 may detect that the initial reorientation of the surgical tool 22 occurs at point P2 and the controller 30 may generate a virtual constraint at point P2.

More particularly, the controller 30 may detect the location of the initial reorientation relative to the tool path 70, relative to the surgical site S, and/or relative to an obstacle to be avoided. In the instance of FIG. 6, the surgical tool 22 moves along the tool path 70 and is reoriented at point P2 along the tool path 70. The controller 30 may detect the location of point P2 relative to the tool path 70. Additionally, the controller 30 may detect the location of point P2 relative to the surgical site S. In instances where the boundary generator 66 has identified an obstacle to be avoided, the controller 30 may record the location of point P2 relative to the obstacle to be avoided. In the instance of FIG. 7, the surgical tool 22 does not move along a tool path 70. As follows, the controller 30 may detect the location of point P2 relative to the surgical site S, as point P2 is a location within the surgical site S. Additionally, in instances where the boundary generator 66 has identified an obstacle to be avoided, the controller 30 may record the location of point P2 relative to the obstacle to be avoided.

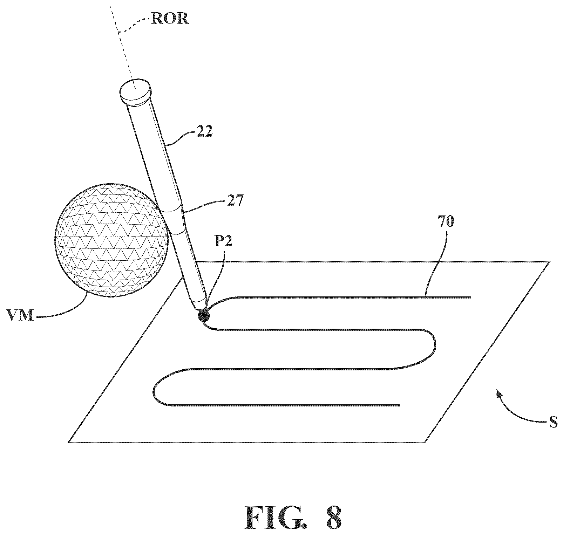

FIGS. 8-10 illustrate instances of step 106 where the controller 30 generates a virtual constraint in response to detecting the initial reorientation of the surgical tool 22. As previously described, FIGS. 6 and 7 illustrate instances where the surgical tool 22 is reoriented at point P2 and where the controller 30 detects the initial reorientation. The retractor R is not shown in FIGS. 8-10 for simplicity.

In the instances of FIGS. 8-10, the initial reorientation occurs at point P2. Accordingly, the controller 30 detects the location of initial reorientation as the location of point P2. Additionally, in the instances of FIGS. 8-10, the controller 30 operates in the automatic, semi-autonomous, or guided-manual mode and moves the surgical tool 22 along a tool path 70. During movement along the tool path 70, the controller 30 generates a virtual constraint at the detected location of initial reorientation, point P2. In other instances, the controller 30 may generate the virtual constraint at the recorded location of initial reorientation while the controller 30 operates in the manual mode.

In the instance of FIG. 8, the controller 30 generates a virtual mesh VM. In instances where the controller 30 generates a virtual mesh, the controller 30 may generate the virtual mesh relative to the recorded location of initial reorientation. In the instance of FIG. 8, the controller 30 generates the virtual mesh VM to be adjacent to the detected location of initial reorientation, point P2. In other instances, the virtual mesh may be generated to be any suitable distance from the detected location of initial reorientation. For example, the virtual mesh VM may be generated to be at the point P2. Additionally, the virtual mesh VM may include any suitable shape and size. For example, in the instances of FIG. 8, the virtual mesh VM includes a spherical shape. In other instances, the virtual mesh VM may include a planar shape of any suitable size or any suitable polygonal shape of any suitable size.

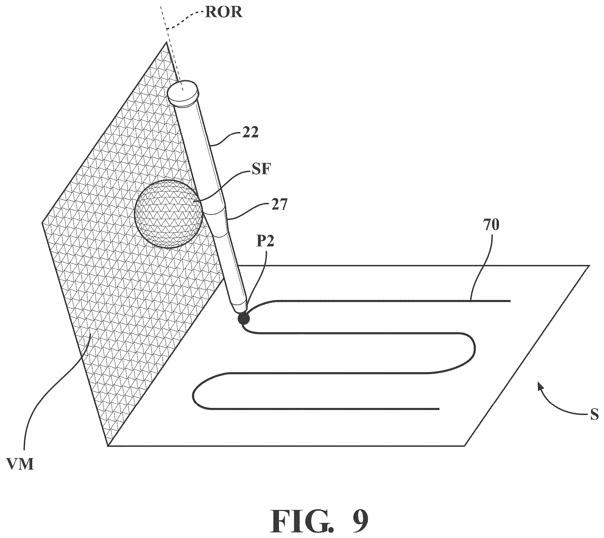

In the instance of FIG. 9, the controller 30 generates a stereotactic interaction feature SF. In instances where the controller 30 generates a stereotactic interaction feature, the controller 30 may generate the stereotactic interaction feature relative to the surgical tool 22, relative to the recorded location of initial reorientation, and/or relative to a virtual constraint. In the instance of FIG. 9, the controller 30 generates the stereotactic interaction feature SF relative to the surgical tool 22. As shown, the stereotactic interaction feature SF contacts a shaft 27 of the surgical tool 22.

In instances where the controller 30 generates a stereotactic interaction feature, the controller 30 may generate more than one stereotactic interaction feature. For example, the controller 30 may generate a stereotactic interaction feature relative to the surgical tool 22, relative to the recorded location of initial reorientation, and/or relative to a virtual constraint. In one such instance, the controller 30 may generate a stereotactic interaction feature relative to the surgical tool 22 and relative to the detected location of initial reorientation.

The stereotactic interaction feature may be generated relative to the surgical tool 22, the detected location of initial reorientation, and/or the virtual constraint such that the stereotactic interaction feature may be any suitable distance from the surgical tool 22, the detected location of initial reorientation, and/or the virtual constraint. For instance, the stereotactic interaction feature SF of FIG. 9 may be generated to be adjacent to an energy applicator of the surgical tool 22, without contacting the surgical tool 22.

The stereotactic interaction feature may include any suitable shape and size. For example, in the instance of FIG. 9, the stereotactic interaction feature SF includes a spherical shape. However, in other instances, the stereotactic interaction feature SF may include any suitable polygonal shape of any suitable size.

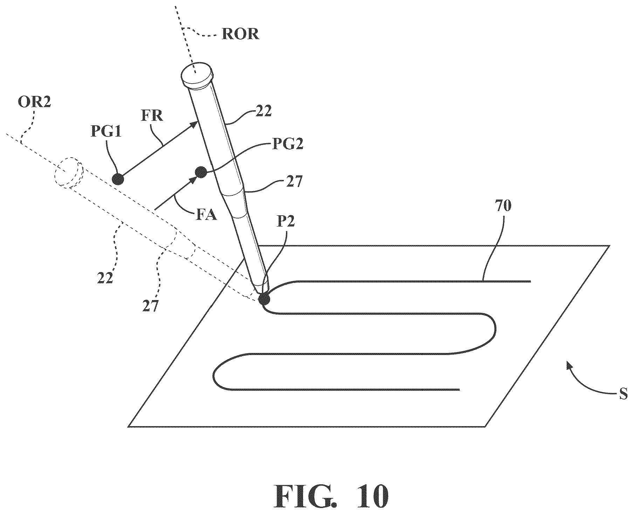

In the instance of FIG. 10, the controller 30 generates a particle guide with a constraint force to be applied to the surgical tool 22. Specifically, in the instance of FIG. 10, the controller 30 is shown illustratively as generating a first particle guide PG1 with a corresponding repulsive force FR and a second particle guide PG2 with a corresponding attractive force FA. The controller 30 may generate any suitable number of particle guides with a corresponding repulsive or attractive force. For example, the controller 30 may generate a single particle guide with a corresponding repulsive force.

In instances where the controller 30 generates a particle guide, the controller 30 may generate the particle guide relative to the detected location of initial reorientation. In the instance of FIG. 10, the controller 30 generates the particle guides PG1, PG2 to be adjacent to the detected location of initial reorientation, point P2. The particle guide may be located any suitable distance from the detected location of initial reorientation. Additionally, the controller 30 may generate the particle guide relative to the detected location of initial reorientation based on a magnitude of the corresponding repulsive or attractive force. For example, the controller 30 may generate the particle guide at a distance relative to the detected location of initial reorientation, where the distance corresponds to a magnitude of the repulsive or attractive force.

The virtual constraints generated by the controller 30 may be activated or deactivated. For example, the virtual constraint generated during step 106 may be activated when the surgical tool 22 is within a proximity of the location of initial reorientation, where the proximity may be any suitable distance. For example, the stereotactic interaction feature SF of FIG. 9 may be activated by the controller when the surgical tool 22 is within a proximity of the detected location of initial reorientation and deactivated when the surgical tool 22 is not within the proximity of the detected location of initial reorientation. Advantageously, in such instances, the virtual constraints generated by the controller 30 interact with the surgical tool 22 when the surgical tool 22 is within the proximity of the detected location of initial reorientation and do not interact with the surgical tool 22 when the surgical tool 22 is not within the proximity of the detected location of initial reorientation. As another example, temporary virtual constraints may be activated or deactivated.

The controller 30 may notify an operator of a generation of a virtual constraint. The controller 30 may provide haptic, audible, and/or visual feedback to an operator of the surgical system 10 in response to generating the virtual constraint. For example, the controller 30 may provide a vibration pattern to one or more of the input/output devices 40, 42, 43 in response to generating the virtual constraint. As another example, the controller 30 may provide a visual notification via a display of surgical system 10 in response to generating the virtual constraint. The controller 30 may present a 2D/3D image of the virtual constraint to provide the visual notification. The display may be the display 38 of the interactive touchscreen devices 40, 42 and/or a display of a head-mounted device. In some instances, the controller 30 may request confirmation of the generation of the virtual constraint from the operator. The controller 30 may request confirmation via a display of the surgical system 10. In instances where the input/output devices 40, 42, 43 include a head-mounted device, the head-mounted device may be configured to receive the confirmation by tracking a gesture, a gaze, and/or a head motion of the operator. The head-mounted device may also include a microphone and/or audio sensor configured to receive voice instructions/commands as the confirmation from the user.

### e. Interaction Between Virtual Constraint and Surgical Tool

During step 108, the virtual constraint generated during step 106 facilitates subsequent reorientation of the surgical tool 22. Specifically, the virtual constraint facilitates subsequent reorientation of the surgical tool 22 at the recorded location in response to interaction between the surgical tool 22 and the virtual constraint during subsequent movement of the surgical tool 22. As previously stated, during subsequent movement of the surgical tool 22, the surgical tool 22 may again be located at the location of detected initial reorientation or near the location of detected initial reorientation. Therefore, once the virtual constraint has been generated during step 106 and the surgical tool 22 is again located at the recorded location of the initial reorientation or near the location of the detected initial reorientation, the generated virtual constraint may interact with the surgical tool 22, reorienting the surgical tool 22.

Generally, the virtual constraint may interact with the surgical tool 22 to cause movement of the surgical tool 22 that emulates the initial reorientation. For example, referring to FIGS. 6 and 7, the surgical tool 22 is initially reoriented to orientation ROR. As follows, the virtual constraint generated during step 106 would interact with the surgical tool 22 to subsequently reorient the surgical tool 22 to orientation ROR. The virtual constraint may reorient the tool by causing movement of the surgical tool 22 that emulates a force applied to the surgical tool 22, a velocity of the surgical tool 22, and/or an acceleration of the surgical tool 22 during the initial reorientation. In one such instance, the virtual constraint may apply a constraint force to the surgical tool 22 based on an external force applied to the surgical tool 22 during the initial reorientation. In particular, the constraint force may emulate a magnitude and direction of the external force applied to the surgical tool 22 during the initial reorientation.

FIGS. 8-10 illustrate instances of step 108 where the virtual constraint generated during step 106 interacts with the surgical tool 22, facilitating the subsequent reorientation of the surgical tool 22. In the instances of FIGS. 8-10, the subsequent reorientation of the surgical tool 22 occurs when the surgical tool 22 is again located at the location of detected initial reorientation, point P2. However, in other instances, the subsequent reorientation of the surgical tool 22 may occur when the surgical tool 22 is near the location of detected initial reorientation.

In the instance of FIG. 8, the virtual mesh VM reorients the surgical tool 22 by contacting the surgical tool 22. Specifically, the virtual mesh VM contacts the surgical tool 22 to reorient the surgical tool 22 to the orientation ROR, emulating the initial reorientation of FIG. 6. During step 106, the virtual mesh may be generated to include any suitable size or shape such that an interaction between the virtual mesh and the surgical tool 22 emulates the initial reorientation. Additionally, the virtual mesh may include any suitable position relative to the detected location of initial reorientation to emulate the initial reorientation.

In the instance of FIG. 9, the stereotactic interaction feature SF reorients the tool by contacting a virtual constraint. Specifically, the stereotactic interaction feature SF contacts a virtual mesh VM to reorient the surgical tool 22 to the orientation ROR, emulating the reorientation of FIG. 6. During step 106, the stereotactic interaction feature may be generated to include any suitable size or shape such that an interaction between the stereotactic interaction feature and a virtual constraint emulates the initial reorientation. Additionally, the stereotactic interaction feature may be generated at any suitable location relative to the surgical tool 22, the detected location of initial reorientation, and/or a virtual constraint to emulate the initial reorientation.

The virtual constraint contacted by the stereotactic interaction feature may be any suitable virtual constraint, such as a virtual mesh and/or another stereotactic interaction feature. Additionally, the virtual constraint contacted by the stereotactic interaction feature may be generated during the method 100 of facilitating reorientation of a surgical tool 22 or prior to execution of the method 100. For example, the stereotactic interaction feature SF and the virtual mesh VM of FIG. 9 may have both been generated during a single instance of step 106. As another example, the stereotactic interaction feature SF may have been generated during an instance of step 106 and the virtual mesh VM may have been generated prior to execution of the method 100.

In the instance of FIG. 10, the particle guides PG1, PG2 reorient the surgical tool 22 by applying the repulsive force FR and the attractive force FA to the surgical tool 22. Specifically, the particle guides PG1, PG2 apply the repulsive force FR and the attractive force FA to the surgical tool 22 to reorient the surgical tool 22 from the orientation OR2 to the orientation ROR, emulating the reorientation of FIG. 6. During step 106, the particle guide may be generated to apply any suitable constraint force to the surgical tool 22 to cause the desired reorientation. Additionally, the particle guide may include any suitable position relative to the detected location of initial reorientation to cause the desired reorientation.

The virtual constraints generated by the controller 30 may be activated or deactivated at any time. For example, the virtual constraint generated during step 106 may be activated when the surgical tool 22 is within a proximity of the location of initial reorientation, where the proximity may be any suitable distance. For example, the stereotactic interaction feature SF of FIG. 9 may be activated by the controller when the surgical tool 22 is within a proximity of the detected location of initial reorientation and deactivated when the surgical tool 22 is not within the proximity of the detected location of initial reorientation. Advantageously, in such instances, the virtual constraints generated by the controller 30 interact with the surgical tool 22 when the surgical tool 22 is within the proximity of the detected location of initial reorientation and do not interact with the surgical tool 22 when the surgical tool 22 is not within the proximity of the detected location of initial reorientation.

The controller 30 may notify an operator of an interaction between the surgical tool 22 and a virtual constraint. The controller 30 may provide haptic, audible, and/or visual feedback to an operator of the surgical system 10 in response to the interaction between the surgical tool 22 and the virtual constraint. For example, the controller 30 may provide a vibration pattern to one or more of the input/output devices 40, 42, 43 in response to the interaction between the surgical tool 22 and a virtual constraint. As another example, the controller 30 may provide a visual notification via a display of surgical system 10 in response to the interaction between the surgical tool 22 and the virtual constraint. The display may be the display 38 of the interactive touchscreen devices 40, 42 and/or a display of a head-mounted device. In some instances, the controller 30 may notify an operator of a potential interaction between the surgical tool 22 and the virtual constraint. For example, the controller 30 may present the operator with the virtual constraints proximate the current location of the surgical tool 22 via a display of the surgical system 10. In an example implementation, the controller 30 may present the operator with the activated temporary virtual constraints proximate the current location of the surgical tool 22 via a display of a head-mounted device.

### f. Other Features

### i. Temporary Virtual Constraints

The controller 30 may be configured to generate temporary virtual constraints. The temporary virtual constraints may be generated prior to or during the method 100. A temporary virtual constraint may be generated in response to detection of the initial reorientation. For example, the controller 30 may be configured to generate a temporary virtual constraint during step 106 in response to detection of the initial reorientation during step 104.

As an example, the controller 30 may be configured to generate a temporary virtual constraint in response to determining that the surgical tool 22 has deviated from the tool path 70 during detection of an initial reorientation. In such an instance, the surgical tool 22 may be blocked from traveling between a first and a second point along the tool path 70 due to an obstacle, such as a retractor R and/or a surgical assistant. The operator may apply external forces/torques to the surgical tool 22 to reorient and move the surgical tool 22 away from the tool path 70 at the first point to avoid the obstacle. The operator may then apply external forces/torques to the surgical tool 22 to move the surgical tool 22 toward the tool path 70 at the second point such that the surgical tool 22 may continue along the tool path 70. Accordingly, the controller 30 may generate a temporary virtual constraint between the first and second points of the tool path 70 to displace the surgical tool 22 from the tool path 70 during subsequent movement of the tool along the tool path. The temporary virtual constraint may be generated to emulate the detected initial reorientation, i.e., the movement of the surgical tool 22 away from and toward the tool path 70.

Generally, a temporary virtual constraint may be defined as a virtual constraint that may be removed/deactivated by the controller 30.

For example, in some instances, the controller 30 may remove/deactivate a temporary virtual constraint after a predetermined amount of time has passed. In an instance where the controller 30 generates a temporary virtual constraint in response to determining that the surgical tool 22 has deviated from the tool path 70, the predetermined amount of time may be a sufficient amount of time for the completion of the surgical procedure.

As another example, the controller 30 may remove/deactivate a temporary virtual constraint in response to completion of a step of a surgical procedure. In an instance where the controller 30 generates a temporary virtual constraint in response to determining that the surgical tool 22 has deviated from the tool path 70, the controller 30 may remove/deactivate the temporary virtual constraint in response to the surgical tool 22 completing movement along the path 70. In such instances, the surgical tool 22 may deviate from the tool path 70 to avoid an obstacle along the tool path 70. The surgical tool 22 may be deviated in a manner that allows the surgical tool 22 to continue along the tool path 70 after the obstacle has been avoided. The controller 30 may remove/deactivate the temporary virtual constraint in response to completion of movement of the surgical tool 22 along the tool path 70. For example, in an instance where the obstacle is a retractor R, the controller 30 may remove/deactivate the temporary virtual constraint in response to the retractor R being removed. As another example, in an instance where the obstacle is an arm of a surgical assistant, the controller 30 may remove/deactivate the temporary virtual constraint in response to the surgical assistant changing position such that the surgical assistant no longer prevents movement along the tool path 70. The controller 30 may determine that an obstacle has been removed based on an input from a user interface of the surgical system 10 (e.g., the operator may interact with a user interface to indicate the removal of an object) and/or based on tracking by the navigation system 32.

As another example, the controller 30 may remove/deactivate a temporary virtual constraint in response to an action by the operator of the surgical system 10. In an instance where the controller 30 generates a temporary virtual constraint in response to determining that the surgical tool 22 has deviated from the tool path 70, the controller 30 may remove/deactivate the temporary virtual constraint in response to detecting that an obstacle that was blocking the tool path 70 has been removed. In such instances, the operator may reorient the surgical tool 22 to avoid an obstacle along the tool path 70 and the controller 30 may generate a temporary virtual constraint in response to the reorientation. Once the obstacle has been removed, the controller 30 may remove/deactivate the temporary virtual constraint.

In some instances, upon removal/deactivation of a temporary virtual constraint, the controller 30 may be configured to generate a new tool path 70 to resect tissue that was previously avoided during reorientation. For example, in an instance where the surgical tool 22 deviates from the tool path 70 to avoid an obstacle along the tool path 70, the controller 30 may generate a new tool path 70 for resecting tissue that was avoided during the deviation. In such an instance, the controller 30 may generate a temporary virtual constraint in response to determining that the surgical tool 22 has deviated from the tool path 70. Once the temporary virtual constraint has been removed, the controller 30 may generate a new tool path 70 to resect the avoided tissue. The controller 30 may then be configured to facilitate movement of the end effector 20 and surgical tool 22 along the new tool path 70 for resecting the avoided tissue.

The controller 30 may notify an operator of a generation of a temporary virtual constraint. The controller 30 may provide haptic, audible, and/or visual feedback to an operator of the surgical system 10 in response to the generation of a temporary virtual constraint. For example, the controller 30 may provide a vibration pattern to one or more of the input/output devices 40, 42, 43 in response to the generation of a temporary virtual constraint. As another example, the controller 30 may provide a visual notification via a display of surgical system 10 in response to the generation of a temporary virtual constraint. The controller 30 may present a 2D/3D image of the generated temporary virtual constraint to provide the visual notification. The display may be the display 38 of the interactive touchscreen devices 40, 42 and/or a display of a head-mounted device. In some instances, the controller 30 may label the generated temporary virtual constraints. For example, a display of the surgical system 10 may provide a menu of the virtual constraints generated by the boundary generator 66. The display may indicate the fixed virtual constraints (e.g., constraints that represent the tracked anatomy of the patient 12) using a first label and indicate the temporary virtual constraints using a second label.

### ii. User Interface

In some implementations, the surgical system 10 may include a user interface configured to receive an input from or provide an output to the operator of the surgical system 10. For example, the operator may provide an input to the user interface to manage the virtual constraints of the surgical system 10. Additionally, the user interface may output notifications and/or 2D/3D images related to the virtual constraints to the operator. The user interface may be any of the previously described input/output devices 40, 42, 43. For instance, the operator may provide an input to one or more of the interactive touchscreen displays 40, 42 to manage the virtual constraints. Additionally, the operator may view notifications and/or 2D/3D images related to the virtual constraints on a display, such as the display 38 of the interactive touchscreen devices 40, 42 and/or a display of a head-mounted device.

In another example implementation, the user interface may be a button located on the surgical tool 22, and the button may be depressed to provide an input to the surgical system 10. For example, the button may be located on the end effector 20 and/or the surgical tool 22. In some instances, the button may be the footswitch 43. The button may also be located on a hand-held pendant device, as shown and described in U.S. Patent No. 9,119,655, entitled, "Surgical Manipulator Capable of Controlling a Surgical Instrument in Multiple Modes," the disclosure of which is hereby incorporated by reference. Depressing the button signals that the operator desires to reorient the surgical tool 22 and the controller 30 may detect a reorientation of the surgical tool 22 based on depression of the button.

The operator may manage the virtual constraints of the surgical system 10 by confirming generation of a virtual constraint. For example, the controller 30 may provide a notification/prompt to an operator related to a generation of a virtual constraint prior to the generation of the virtual constraint during step 106. In such instances, the operator may confirm generation of the virtual constraint by providing an input to the user interface in response to the notification/prompt. Similarly, the operator may reject generation of the virtual constraint by providing an input to the user interface in response to the prompt. In some instances, the controller 30 may assume that the operator approves generation of virtual constraints and may provide a notification that a virtual constraint has been generated.

The operator may manage the virtual constraints of the surgical system 10 by modifying properties of the virtual constraints. For example, the operator may modify a size and/or a shape of a virtual mesh and/or a stereotactic interaction feature by providing an input to the user interface. As another example, the operator may modify a direction and/or magnitude of a constraint force generated by a particle guide. The operator may also modify a position of a virtual mesh and/or a particle guide relative to the detected location of initial reorientation, and the operator may modify a position of the stereotactic interaction feature relative to the surgical tool 22. The operator may also merge more than one virtual constraint into a single virtual constraint or separate a virtual constraint into more than one virtual constraint. In one such instance, the user interface may provide a menu of the virtual constraints on a display of the user interface and the operator may modify a property of a virtual mesh, a stereotactic interaction feature, and/or a particle guide by interacting with the user interface.

The operator may manage the virtual constraints of the surgical system 10 by removing virtual constraint(s) and/or adding additional virtual constraints. Similarly, the operator may manage the virtual constraints of the surgical system 10 by deactivating/activating virtual constraint(s). For example, the operator may remove/deactivate/activate a virtual mesh, a stereotactic interaction feature, and/or a particle guide and/or add an additional virtual mesh, a stereotactic interaction feature, and/or a particle guide including any suitable size/shape at any suitable location. In one such instance, the user interface may provide a menu of the virtual constraints on a display of the user interface and the operator may remove/activate/deactivate/add a virtual mesh, a stereotactic interaction feature, and/or a particle guide by interacting with the user interface.

The operator may manage virtual constraints generated during the method 100 of facilitating reorientation of a surgical tool 22 and/or virtual constraints generated prior to execution of the method 100. For instance, the operator may modify and/or remove virtual constraints that were generated during the step 106 of generating a virtual constraint. The operator may also modify and/or remove virtual constraints that were generated prior to the step 102 of controlling the manipulator 14 to facilitate movement of the surgical tool 22.

### iii. Mobility-based Virtual Constraints

As previously stated, the virtual constraints of the system may be generated based on a mobility of the operator and/or a preference of the operator. In such instances, the surgical tool 22 may be reoriented to allow the surgical tool 22 to be moved in accordance with the desires and/or ability of the operator. Reorientation of the surgical tool 22 may allow the surgical tool 22 to be moved in a manner that is more ergonomically friendly to an operator. For example, in the guided-manual mode, the controller 30 may constrain the surgical tool 22 such that the surgical tool 22 moves along the tool path 70 in preferred orientations in response to external forces/torques applied to a component of the manipulator 14. However, in some instances, such preferred orientations may not be ergonomically friendly as they may put unnecessary stress on the operator. As such, the operator may apply external forces/torques to move the surgical tool 22 to an orientation that is more ergonomically friendly, causing the initial reorientation. Accordingly, the virtual constraint generated during step 106 may emulate the initial reorientation, facilitating the ergonomically friendly orientation during subsequent movement of the surgical tool 22. As another example, the operator may have a preferred wrist positioning and/or hand preference (e.g., left hand or right hand) for grasping and applying external forces/torques to the end effector 20. In such instances, the operator may apply external forces/torques to move the surgical tool 22 to an orientation that allows the operator to grasp the end effector 20 and/or surgical tool 22 using the preferred wrist position and/or hand preference, causing the initial reorientation. Accordingly, the virtual constraint generated during step 106 may emulate the initial reorientation, facilitating the preferred orientation during subsequent movement of the surgical tool 22.

The virtual constraints of the system may also be generated based on a mobility of the manipulator 14. In such instances, the surgical tool 22 may be reoriented to allow the manipulator 14 to move the surgical tool 22 without restriction. For example, the controller 30 may be prevented from moving along the tool path 70 in a preferred orientation due to one or more of a workspace boundary of the manipulator 14, a range of motion of the manipulator 14, a limit of a joint (J) of the manipulator 14, and a singularity of the manipulator 14. For instance, the range of motion of the manipulator 14 may be limited at a point along the tool path 70, preventing the surgical tool 22 from achieving a preferred orientation at the point. In such instances, the surgical tool 22 may be reoriented to allow the manipulator 14 to continue movement along the tool path 70, despite a limitation in the range of motion of the manipulator 14 at the point along the tool path 70. Furthermore, a virtual constraint generated during step 106 may facilitate the subsequent reorientation of the surgical tool 22 based on one or more of a workspace boundary of the manipulator 14, a range of motion of the manipulator 14, a limit of a joint (J) of the manipulator 14, and a singularity of the manipulator 14. For example, the virtual constraint may apply a constraint force to reorient the surgical tool 22 toward the base 16 of the manipulator 14 such that the manipulator 14 remains within the workspace boundary. As another example, the virtual constraint may apply a constraint force to reorient the surgical tool 22, wherein the constraint force does not cause the robotic arm 18A to exceed a range of motion of the manipulator 14. As another example, the virtual constraint may apply a constraint force to reorient the surgical tool 22, wherein the constraint force does not cause the robotic arm 18A to position the manipulator 14 at a singularity, where degrees of freedom (DOF) the manipulator 14 are reduced.

### iv. Automatic Modification of Virtual Constraints

In some implementations, the controller 30 may automatically modify a previously generated virtual constraint. The virtual constraint may be any suitable type of virtual constraint, such as a virtual mesh, a stereotactic interaction feature, and/or a particle guide. The controller 30 may modify the previously generated virtual constraint by modifying a parameter of the virtual constraint, such as a constraint force, a size, a shape, and/or a location of the virtual constraint. The previously generated virtual constraint may be a temporary virtual constraint.

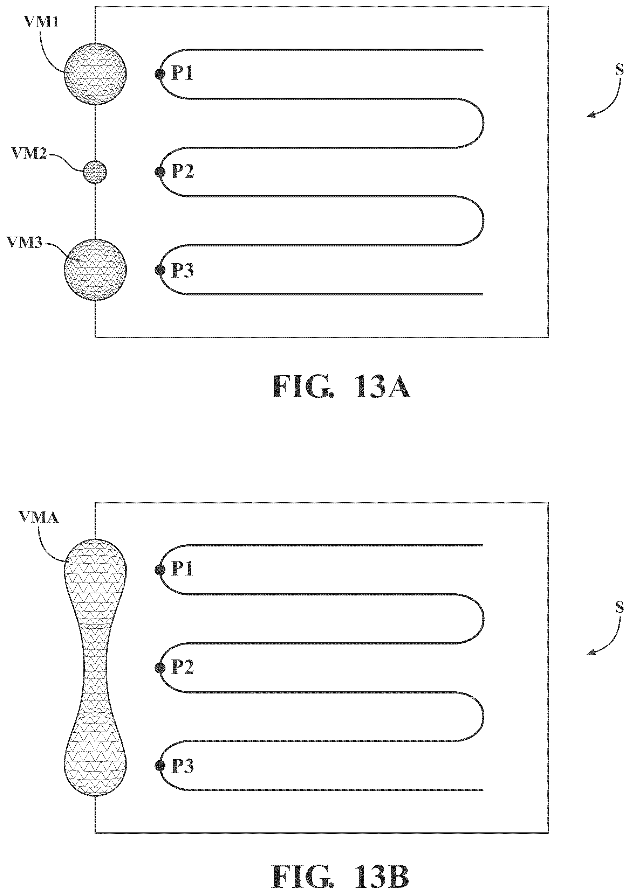

In one such instance, the controller 30 may automatically merge several virtual constraints into a single virtual constraint. For example, referring to FIG. 13A, the controller 30 has generated a first virtual constraint VM1 in response to detecting a first initial reorientation at point P1, a second virtual constraint VM2 in response to detecting a second initial reorientation at point P2, and a third virtual constraint VM3 in response to detecting a third initial reorientation at point P3. As shown in FIG. 13B, the controller 30 may automatically aggregate the first, second, and third virtual constraints VM1, VM2, VM3 into a single aggregate virtual constraint VMA.

In another instance, the controller 30 may automatically modify a previously generated virtual constraint in response to detecting a reorientation of the surgical tool 22. Such a reorientation may be referred to herein as a modifying reorientation. In such an instance, the modifying reorientation causes a modifying interaction with the previously generated virtual constraint and the controller 30 modifies the previously generated virtual constraint based on the modifying interaction. A modifying interaction may be defined as an interaction between the surgical tool 22 and the previously generated virtual constraint, which differs from an expected interaction between the surgical tool 22 and the previously generated virtual constraint. For example, the controller 30 may anticipate that the surgical tool 22 will apply an expected amount of force to the virtual constraint during the expected interaction. During the modifying interaction, the surgical tool 22 may apply a different amount of force to the virtual constraint than the expected amount of force.

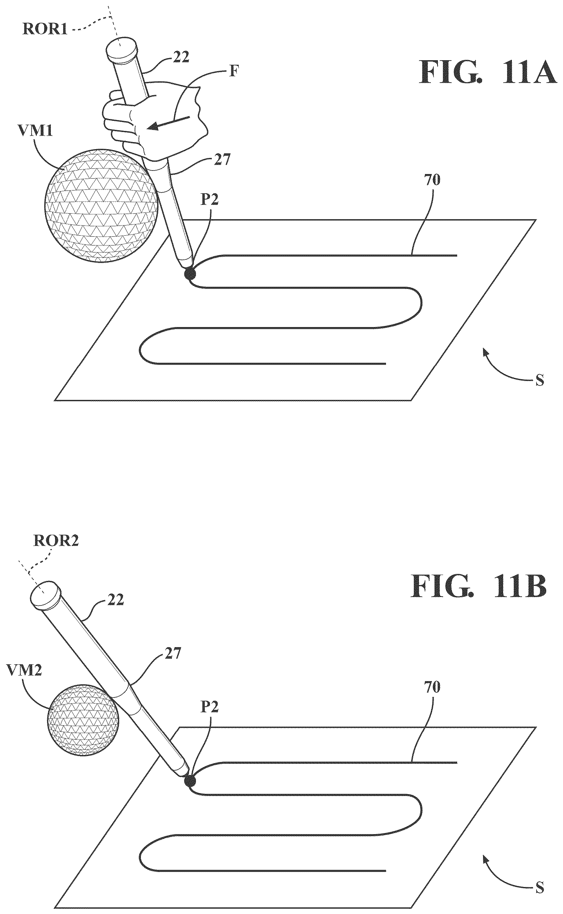

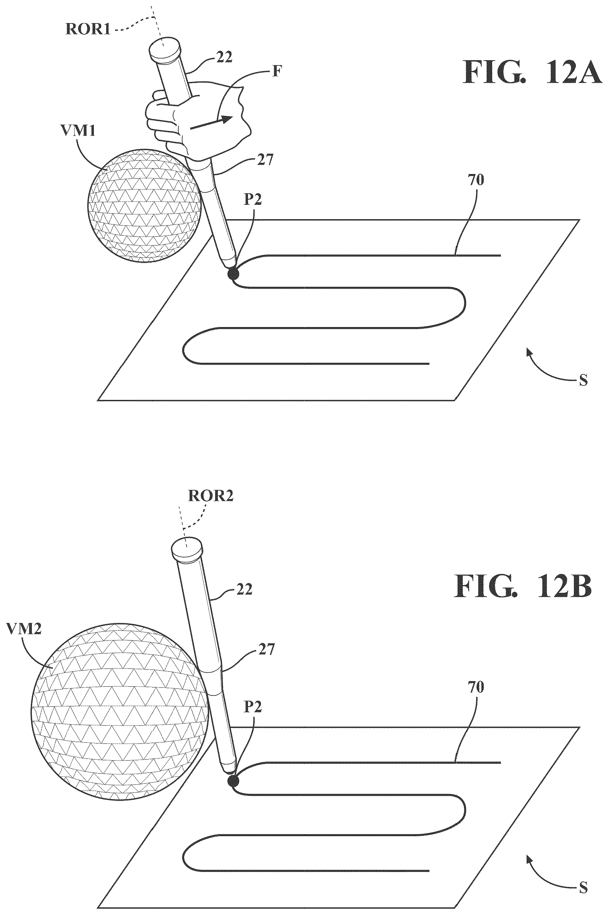

FIGS. 11A-12B illustrate instances where the controller 30 automatically modifies a size of a virtual mesh VM. In both FIGS. 11A and 12A, the controller 30 has generated the virtual mesh VM in response to detecting an initial reorientation of the surgical tool 22 to orientation ROR1 at the location of detected reorientation, point P2. As shown, the virtual mesh VM of FIGS. 11A and 12A interacts with the surgical tool 22 to facilitate subsequent reorientation of the surgical tool 22 to orientation ROR1.

In FIG. 11B, the controller 30 has reduced a size of the virtual mesh VM in response to detecting a modifying reorientation of the surgical tool 22. The modifying reorientation of the surgical tool 22 is shown in FIG. 11A, where an operator applies an external force F to the surgical tool 22 that is directed toward the virtual mesh VM. The controller 30 may determine that, due to the application of the external force F, the interaction between the surgical tool 22 and the virtual mesh VM is a modifying interaction that differs from the expected interaction between the surgical tool 22 and the virtual mesh VM. Additionally, the controller 30 may determine that, due to the direction of the external force F being toward the virtual mesh VM, the operator may want to reduce a size of the virtual mesh VM. FIG. 11B illustrates a second subsequent reorientation of the surgical tool 22, where the virtual mesh VM reorients the surgical tool 22 to orientation ROR2, instead of orientation ROR1, due to the reduced size of the virtual mesh VM. In the instance of FIG. 11B, the controller 30 may modify the size of the virtual mesh VM based on the external force F. For example, the reduction in size of the virtual mesh VM may be proportional to a magnitude of the external force F.

In FIG. 12B, the controller 30 has increased a size of the virtual mesh VM in response to detecting a second initial reorientation of the surgical tool 22. The second initial reorientation of the surgical tool 22 is shown in FIG. 12A, where an operator applies an external force F to the surgical tool 22 that is directed away from the virtual mesh VM. The controller 30 may determine that, due to the application of the external force F, the interaction between the surgical tool 22 and the virtual mesh VM is a modifying interaction that differs from the expected interaction between the surgical tool 22 and the virtual mesh VM. Additionally, the controller 30 may determine that, due to the direction of the external force F being away from the virtual mesh VM, the operator may want to increase a size of the virtual mesh VM. FIG. 12B illustrates a second subsequent reorientation of the surgical tool 22, where the virtual mesh VM reorients the surgical tool 22 to orientation ROR2, instead of orientation ROR1, due to the increased size of the virtual mesh VM. In the instance of FIG. 12B, the controller 30 may modify the size of the virtual mesh VM based on the external force F. For example, the increase in size of the virtual mesh VM may be proportional to a magnitude of the external force F.

The controller 30 may also remove/deactivate a virtual constraint based on a modifying reorientation. For example, the controller 30 may remove/deactivate/activate a virtual mesh, a stereotactic interaction feature, and/or a particle guide in response to detecting an external force/torque applied to the surgical tool 22 that is directed toward the virtual mesh, a stereotactic interaction feature, and/or a particle guide.

The modifying reorientation may be triggered by an input from an operator. For example, the modifying reorientation may be triggered in response to the user interacting with a user interface (e.g., a button located on the surgical tool 22).

The modifying reorientation may occur at any suitable location. As previously stated, the modifying reorientation causes a modifying interaction with the previously generated virtual constraint. As such, the modifying reorientation may occur at any location where a modifying interaction may occur. For example, in instances where the virtual constraint was generated at a detected location of initial reorientation, the modifying reorientation may occur at the detected location of initial reorientation and/or near the detected location of initial reorientation during subsequent movement of the surgical tool 22.

The previously generated virtual constraint may be generated prior to execution of the method 100 and/or during the method 100. In instances where a virtual constraint was generated prior to execution of the method 100, the virtual constraint may be modified in response to detecting an initial reorientation of the surgical tool 22 during step 104. In other words, instead of generating a new virtual constraint during step 106 in response to detecting the initial reorientation during step 104, the controller 30 may modify the existing virtual constraint. In instances where the virtual constraint was generated during the step 106 in response to detecting an initial reorientation, the controller 30 may modify the generated virtual constraint in response to detecting a second initial reorientation of the surgical tool 22. In other words, since a virtual constraint was generated during step 106 in response to detecting an initial reorientation during step 104, the controller 30 modifies the generated virtual constraint in response to detecting a second initial reorientation.

In some instances, the controller may generate a new virtual constraint in response to a modifying reorientation. In one such instance, the controller 30 may generate a new virtual constraint without removing or modifying the previously generated virtual constraint. The newly generated virtual constraint may be of the same or different type as the previously generated virtual constraint. For example, the controller 30 may generate a stereotactic interaction feature in response to a modifying interaction between the surgical tool 22 and a previously generated virtual mesh, where the stereotactic interaction feature may interact with the previously generated virtual mesh during subsequent movement of the surgical tool 22. In another instance, the controller 30 may replace the previously generated virtual constraint with a newly generated virtual constraint. The replacing virtual constraint may be of the same or different type as the previously generated virtual constraint. For example, the controller 30 may replace a previously generated virtual mesh with a particle guide in response to a modifying interaction between the surgical tool 22 and a previously generated virtual mesh, where the particle guide may apply a repulsive force to the surgical tool 22 during subsequent movement of the surgical tool 22.

The controller 30 may notify an operator of an automatic modification of a virtual constraint. The controller 30 may provide haptic, audible, and/or visual feedback to an operator of the surgical system 10 in response to the automatic modification. For example, the controller 30 may provide a vibration pattern to one or more of the input/output devices 40, 42, 43 in response to automatically modifying the virtual constraint. As another example, the controller 30 may provide a visual notification via a display of surgical system 10 in response to automatically modifying the virtual constraint. The controller 30 may present a 2D/3D image of the modified virtual constraint to provide the visual notification. The display may be the display 38 of the interactive touchscreen devices 40, 42 and/or a display of a head-mounted device. In some instances, the controller 30 may request confirmation of the automatic modification of the virtual constraint from the operator. The controller 30 may request confirmation via a display of the surgical system 10. In instances where the input/output devices 40, 42, 43 include a head-mounted device, the head-mounted device may be configured to receive the confirmation by tracking a gesture, a gaze, and/or a head motion of the operator. The head-mounted device may also include a microphone and/or audio sensor configured to receive voice instructions/commands as the confirmation from the user.

### v. Automatic Generation of Virtual Constraints

In some implementations, the controller 30 may generate more than one virtual constraint in response to detecting an initial reorientation of the surgical tool 22 during step 104. Specifically, the controller 30 may generate a virtual constraint relative to a first location in response to detecting the initial reorientation at a first location, and the controller 30 may automatically generate second virtual constraint relative to a second location in response to detecting the initial reorientation at the first location. Advantageously, in such instances, the controller 30 attempts to predict when future reorientations are needed or desired, based on past reorientations.

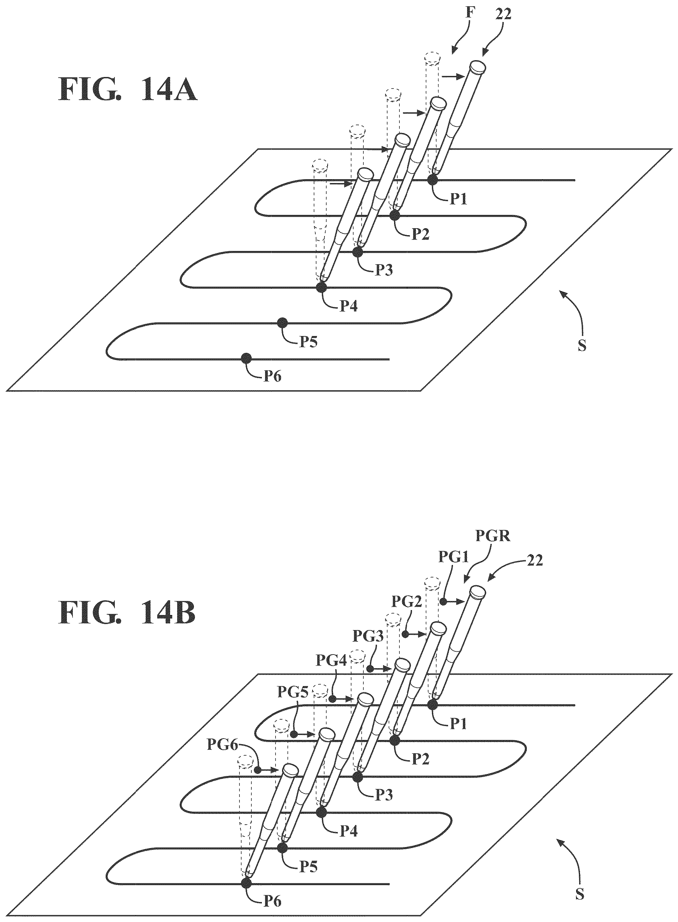

For example, in the instance of FIGS. 14A and 14B, an operator applies an external force F to the surgical tool 22 at each of the points P1-P4, causing a first, second, third, and fourth initial reorientation at points P1-P4. In such an instance, the operator may be applying the external force F due to a limit in the range of motion of the manipulator 14 at each of the points P1-P4. The controller 30 may then attempt to predict future reorientations at points P5 and P6 based on the first, second, third, and fourth initial reorientation at points P1-P4. As shown in FIG. 14B, the controller 30 generates a particle guide PG1-PG4 in response to each of the first, second, third, and fourth reorientations, the particle guides PG1- PG4 including a repulsive force to emulate the first, second, third, and fourth reorientations. Additionally, the controller 30 automatically generates particle guides PG5, PG6 to facilitate reorientation of the surgical tool 22 at points P5 and P6. In this way, once the surgical tool 22 reaches points P5 and P6, the particle guides PG5, PG6 facilitate reorientation of the surgical tool 22, without requiring the operator to provide an external force.

The controller 30 may generate any type of virtual constraint for the automatic generation. For example, the controller 30 may generate a virtual mesh, a stereotactic interaction feature, and/or a particle guide. Additionally, the controller 30 may automatically generate any combination of number of virtual constraints at any suitable location. For example, the controller 30 may automatically generate any combination of one or more virtual meshes, one or more particle guides, and/or one or more stereotactic interaction features at any suitable location. Furthermore, the controller 30 may generate temporary virtual constraints during the automatic generation.

The controller 30 may automatically generate any suitable number of virtual constraints. For example, in the instance of FIGS. 14A-14B, the controller 30 automatically generates two virtual constraints, particle guides PG5, PG6. The number of automatically generated virtual constraints may correspond to a generation sensitivity parameter of the controller 30. For example, the greater the value of the generation sensitivity parameter, the greater the number of virtual constraints that may be automatically generated by the controller 30 and the lower the value of the generation sensitivity parameter, the fewer the number of virtual constraints that may be automatically generated by the controller 30. The generation sensitivity parameter of the controller may be adjusted by an operator via a user interface of the system 10.

The controller 30 may automatically generate a virtual constraint in response to any suitable number of initial reorientations. For example, in the instance of FIGS. 14A-14B, the controller 30 automatically generates particle guides PG5, PG6 in response to detecting four initial reorientations, the first, second, third, and fourth initial reorientation. In other instances, the controller 30 may automatically generate a virtual constraint based on a single initial reorientation. The number of initial reorientations to be detected by the controller 30 prior to automatic generation of a virtual constraint may correspond to a learning sensitivity parameter of the controller 30. For example, the greater the value of the learning sensitivity parameter, the greater the number of initial reorientations may need to be detected by the controller 30 prior to automatic generation of a virtual constraint. The lower the value of the learning sensitivity parameter, the lesser the number of initial reorientations may need to be detected by the controller 30 prior to automatic generation of a virtual constraint.

The controller 30 may notify an operator of an automatic generation of a virtual constraint. The controller 30 may provide haptic, audible, and/or visual feedback to an operator of the surgical system 10 in response to the automatic generation. For example, the controller 30 may provide a vibration pattern to one or more of the input/output devices 40, 42, 43 in response to automatically generating the virtual constraint. As another example, the controller 30 may provide a visual notification via a display of surgical system 10 in response to automatically generating the virtual constraint. The display may be the display 38 of the interactive touchscreen devices 40, 42 and/or a display of a head-mounted device. In some instances, the controller 30 may request confirmation of the automatic generating of the virtual constraint from the operator. The controller 30 may request confirmation via a display of the surgical system 10. In instances where the input/output devices 40, 42, 43 include a head-mounted device, the head-mounted device may be configured to receive the confirmation by tracking a gesture, a gaze, and/or a head motion of the operator. The head-mounted device may also include a microphone and/or audio sensor configured to receive voice instructions/commands as the confirmation from the user.

As will be understood from the above-description and figures, the techniques described herein provide significant technical solutions over conventional methods. By generating a virtual constraint that facilitates re-orientation of the surgical tool, the techniques avoid the need for a surgeon to manually perform repeated re-orientations or back-and-forth reorientations. The techniques described herein can learn about the obstacle environment from past re-orientation(s) and/or can adaptively predict when a future re-orientation will be required. The solutions provided make the robotic procedure less cumbersome for the surgeon and can alleviate hand or arm fatigue for the surgeon. The solutions reduce delays in the surgical procedure by avoiding the surgeon having to perform manual re-orientations. The solutions provided herein can reduce time pressure, surgeon anxiety and human error involved with manual re-orientations. Moreover, the techniques described herein can improve surgeon ergonomics. For instance, the virtual constraints can reactively or proactively adapt to provide re-orientation of the tool to maintain comfortable wrist positioning for the surgeon.

Any computer or controller referenced herein, unless otherwise described, includes memory, storage, central processing unit (CPU) with one or more processors or microprocessors, input/output devices, etc. The term "memory" is intended to include non-transitory memory associated with a processor or CPU, such as, for example, RAM (random access memory), ROM (read only memory), a fixed memory device (for example, hard drive), a removable memory device (for example, diskette), a flash memory and the like.

As will be appreciated by one skilled in the art, aspects of the present invention may take the form of a computer program product embodied in one or more computer readable medium(s) having computer readable program code embodied thereon. Computer software including instructions or code for performing the methodologies of the invention, as described herein, may be stored in one or more of the associated memory devices (for example, ROM, fixed or removable memory) and, when ready to be utilized, loaded in part or in whole (for example, into RAM) and implemented by a CPU. Such software could include, but is not limited to, firmware, resident software, microcode, and the like.

Several embodiments have been discussed in the foregoing description. However, the embodiments discussed herein are not intended to be exhaustive or limit the invention to any particular form. The terminology which has been used is intended to be in the nature of words of description rather than of limitation. Many modifications and variations are possible in light of the above teachings, and the invention may be practiced otherwise than as specifically described.

The present disclosure also comprises the following clauses, with specific features laid out in dependent clauses, which may specifically be implemented as described in greater detail with reference to the configurations and drawings above:

### CLAUSES

C1. A robotic surgical system comprising: a tool configured to manipulate a surgical site; a manipulator configured to support and move the tool; and one or more controllers configured to: obtain a tool path defined relative to the surgical site; predictively detect, relative to the tool path, a location at which the tool should be re-orientated; generate one or more virtual constraints that are configured to cause the manipulator to reorient the tool at the predictively detected location; and control the manipulator to facilitate movement of the tool along the tool path for treating the surgical site, and in response to the tool reaching the predictively detected location, utilize the one or more virtual constraints to cause the manipulator to re-orient the tool.

C2. The robotic surgical system of clause C1, further comprising a camera that is configured to identify an obstacle or sensitive area relative to the tool path, and wherein the one or more controllers predictively detect the location based on identification of the obstacle or sensitive area by the camera.

C3. The robotic surgical system of clause C2, wherein the camera is attached to the manipulator.

C4. The robotic surgical system of any one of clauses C1-C3, wherein the one or more controllers predictively detect the location based on detection of one or more prior re-orientations of the tool.

C5. The robotic surgical system of any one of clauses C1-C4, wherein the one or more controllers predictively detect the location based on detection of one or more prior detected parameters of the tool, wherein the one or more prior detected parameters include one or more of: a displacement of the tool, a direction of force applied to the tool, a magnitude of force applied to the tool, a velocity of the tool, and an acceleration of the tool.

C6. The robotic surgical system of any one of clauses C1-C5, wherein the one or more controllers predictively detect the location by utilizing a machine learning model that is trained on prior surgical plan data and prior manipulator data.

C7. The robotic surgical system of any one of clauses C1-C6, wherein the one or more controllers predictively customize a feature of the one or more virtual constraints, wherein the predictively customized feature includes one or more of: a location of the one or more virtual constraints, a geometry of the one or more virtual constraints, and/or a stiffness/damping parameter of the one or more virtual constraints.

C8. A method of operating the robotic surgical system of any one of clauses C1-C7.

C1A. A robotic surgical system comprising: a tool configured to manipulate a surgical site; a manipulator configured to support and move the tool; and one or more controllers configured to: control the manipulator to facilitate movement of the tool along a tool path for treating the surgical site; during movement of the tool along the tool path, detect a reorientation of the tool and record a location at which the reorientation occurred; and generate feedback that conveys that a re-orientation is needed, wherein the feedback is configured to be delivered in response to the tool revisiting the recorded location.

C2A. The robotic surgical system of clause C1A, wherein the feedback is haptic feedback provided by the manipulator.

C3A. The robotic surgical system of any one of clauses C1A-C2A, wherein the feedback is visual feedback provided by a light indicator provided on the tool.

C4A. The robotic surgical system of any one of clauses C1A-C3A, wherein the feedback is visual and provided on a display that also presents a representation of the tool, the tool path, and the surgical site.

C5A. The robotic surgical system of clause C4A, wherein the feedback includes a visual identification of the location relative to the representation of the tool path on the display.

C6A. The robotic surgical system of any one of clauses C1A-C5A, wherein the feedback includes a text message or verbal warning that conveys that the re-orientation is needed.

C7A. A method of operating the robotic surgical system of any one of clauses C1A-C6A.

C1B. A robotic surgical system comprising: a tool configured to manipulate a surgical site; a manipulator configured to support and move the tool; and one or more controllers configured to: obtain a tool path defined relative to the surgical site; predictively detect, relative to the tool path, a location at which the tool should be re-orientated; and generate feedback that conveys that a re-orientation is needed, wherein the feedback is configured to be delivered in response to the tool reaching the predictively detected location.

C2B. The robotic surgical system of clause C1B, further comprising a camera that is configured to identify an obstacle or sensitive area relative to the tool path, and wherein the one or more controllers predictively detect the location based on identification of the obstacle or sensitive area by the camera.

C3B. The robotic surgical system of clause C2B, wherein the camera is attached to the manipulator.

C4B. The robotic surgical system of any one of clauses C1B-C3B, wherein the one or more controllers predictively detect the location based on detection of one or more prior re-orientations of the tool.

C5B. The robotic surgical system of any one of clauses C1B-C4B, wherein the one or more controllers predictively detect the location based on detection of one or more prior detected parameters of the tool, wherein the one or more prior detected parameters include one or more of: a displacement of the tool, a direction of force applied to the tool, a magnitude of force applied to the tool, a velocity of the tool, and an acceleration of the tool.

C6B. The robotic surgical system of any one of clauses C1B-C5B, wherein the one or more controllers predictively detect the location by utilizing a machine learning model that is trained on prior surgical plan data and prior manipulator data.

C7B. A method of operating the robotic surgical system of any one of clauses C1B-C6B.

C1C. A robotic surgical system comprising: a tool configured to manipulate a surgical site; a manipulator configured to support and move the tool along a predetermined tool path; a user input device; and one or more controllers configured to: receive input from the input device that identifies one or more locations relative to the predetermined tool path, wherein the one or more locations signify where the user desires a reorientation of the tool to occur; generate one or more virtual constraints that are configured to cause the manipulator to reorient the tool in response to the tool reaching each of the one or more identified locations; control the manipulator to facilitate movement of the tool along the predetermined tool path for treating the surgical site and utilize the one or more virtual constraints to automatically reorient the tool in response to the tool reaching each of the one or more identified locations. C2C. The robotic surgical system of clause C1C, wherein the one or more controllers receive input prior to facilitating movement of the tool along the predetermined tool path.

C3C. The robotic surgical system of clause C1C, wherein the one or more controllers receive input during movement of the tool along the predetermined tool path.

C4C. The robotic surgical system of any one of clauses C1C to C3C, wherein the input device comprises a display that is configured to present a representation of the predetermined tool path and wherein the one or more locations are presented on the display relative to the predetermined tool path.

C5C. The robotic surgical system of any one of clauses C1C to C3C, wherein the input device comprises a button located on the tool or a footswitch connected to the manipulator.

C6C. A method of operating the robotic surgical system of any one of clauses C1C to C5C.

## Claims

1. A robotic surgical system (10) comprising:
a tool (22) configured to manipulate a surgical site (S);
a manipulator (14) configured to support and move the tool (22); and one or more controllers (30, 60, 62) configured to:
control the manipulator (14) to facilitate movement of the tool (22) along a tool path (70) for treating the surgical site (S);
during movement of the tool (22) along the tool path (70), detect a reorientation of the tool (22) and record a location at which the reorientation occurred; and
generate one or more virtual constraints (VM, SF, PG, FA, FR) that are configured to cause the manipulator (14) to reorient the tool (22) in response to the tool (22) revisiting the recorded location.

2. The robotic surgical system (10) of claim 1, wherein the one or more controllers (30, 60, 62) are further configured to generate at least one virtual constraint (VM, SF, PG, FA, FR): at the recorded location; relative to the tool (22); or at the recorded location and relative to the tool (22).

3. The robotic surgical system (10) of any preceding claim, wherein the one or more controllers (30, 60, 62) are configured to customize a feature of the one or more virtual constraints (VM, SF, PG, FA, FR) based on the recorded location relative to the tool path (70), wherein the customized feature includes one or more of: a location of the one or more virtual constraints (VM, SF, PG, FA, FR), a geometry of the one or more virtual constraints (VM, SF, PG, FA, FR), and a stiffness/damping parameter of the one or more virtual constraints (VM, SF, PG, FA, FR).

4. The robotic surgical system (10) of any preceding claim, further comprising a camera that is configured to detect an obstacle or a sensitive area at the recorded location; and wherein the one or more controllers (30, 60, 62) are configured to customize a feature of the one or more virtual constraints (VM, SF, PG, FA, FR) based on the detected obstacle or sensitive area, wherein the customized feature includes one or more of: a location of the one or more virtual constraints (VM, SF, PG, FA, FR), a geometry of the one or more virtual constraints (VM, SF, PG, FA, FR), and a stiffness/damping parameter of the one or more virtual constraints (VM, SF, PG, FA, FR).

5. The robotic surgical system (10) of any preceding claim, wherein:
the one or more controllers (30, 60, 62) are configured to:
control the manipulator (14) to facilitate automatic movement of the tool (22) along the tool path (70) for treating the surgical site (S);
automatically generate the one or more virtual constraints (VM, SF, PG, FA, FR); and
automatically cause the manipulator (14) to reorient the tool (22) in response to the tool (22) revisiting the recorded location;
and optionally, wherein:
the tool (22) is in an original pose prior to being reoriented by the one or more virtual constraints (VM, SF, PG, FA, FR); and
the one or more controllers (30, 60, 62) automatically reorient the tool (22) back to the original pose after the tool (22) passes the recorded location.

6. The robotic surgical system (10) of any preceding claim, wherein the one or more controllers (30, 60, 62) are configured to automatically:
detect a condition wherein the tool (22) no longer needs to revisit the recorded location; and
remove or deactivate the one or more virtual constraints (VM, SF, PG, FA, FR) in response to detection of the condition.

7. The robotic surgical system (10) of any preceding claim, wherein the one or more controllers (30, 60, 62) are configured to detect a parameter of the tool (22) during, or after, occurrence of the reorientation, wherein the detected parameter includes one or more of: a displacement of the tool (22), a direction of force applied to the tool (22), a magnitude of force applied to the tool (22), a velocity of the tool (22), and an acceleration of the tool (22).

8. The robotic surgical system (10) of claim 7, wherein the one or more controllers (30, 60, 62) are configured to customize a feature of the one or more virtual constraints (VM, SF, PG, FA, FR) based on the detected parameter of the tool (22), wherein the customized feature includes one or more of: a location of the one or more virtual constraints (VM, SF, PG, FA, FR), a geometry of the one or more virtual constraints (VM, SF, PG, FA, FR), and/or a stiffness/damping parameter of the one or more virtual constraints (VM, SF, PG, FA, FR).

9. The robotic surgical system (10) of any one of claims 7 to 8, wherein the one or more controllers (30, 60, 62) are configured to:
compare the detected parameter of the tool (22) to a threshold value;
in response to the detected parameter satisfying the threshold value, determine that the reorientation of the tool (22) is intentional and responsive to an external force (F) manually applied to the tool (22) by a user; and
record the location at which the reorientation occurred only in response to determining that the reorientation of the tool (22) is intentional.

10. The robotic surgical system (10) of any preceding claim, wherein:
the tool (22) includes a user interface configured to receive an input to initiate re-orientation of the tool (22); and
in response to receipt of the input from the user interface, the one or more controllers (30, 60, 62) detect the reorientation and record the location at which the reorientation occurred.

11. The robotic surgical system (10) of any preceding claim, wherein:
the one or more virtual constraints (VM, SF, PG, FA, FR) comprise a virtual mesh (VM) defined at the recorded location; and
the one or more controllers (30, 60, 62) cause the manipulator (14) to reorient the tool (22) in response to interaction between the tool (22) and the virtual mesh (VM).

12. The robotic surgical system (10) of claim 11, wherein:
the one or more virtual constraints (VM, SF, PG, FA, FR) further comprise at least one stereotactic interaction feature (SF) defined relative to the tool (22); and
the one or more controllers (30, 60, 62) cause the manipulator (14) to reorient the tool (22) in response to interaction between the at least one stereotactic interaction feature (SF) and the virtual mesh (VM)
and optionally,
the tool (22) comprises a tool shaft (27); and
the at least one stereotactic interaction feature is defined relative to the tool shaft (27).

13. The robotic surgical system (10) of any preceding claim, wherein:
the one or more virtual constraints (VM, SF, PG, FA, FR) comprise an attractive force (FA) or a repulsive force (FR) defined in proximity to the recorded location; and
the one or more controllers (30, 60, 62) cause the manipulator (14) to reorient the tool (22) in response to the tool (22) experiencing the attractive force (FA) or the repulsive force (FR).

14. The robotic surgical system (10) of any preceding claim, wherein the one or more controllers (30, 60, 62) are configured to modify a feature of the one or more virtual constraints (VM, SF, PG, FA, FR) in response to detection of a second reorientation of the tool (22) that occurs at a second location proximate to the recorded location.

15. A method (100) for operating a robotic surgical system (10), the robotic surgical system (10) including a tool (22) configured to manipulate a surgical site (S), a manipulator (14) configured to support and move the tool (22), and one or more controllers (30, 60, 62) for performing the following steps:
controlling (102) the manipulator (14) for facilitating movement of the tool (22) along a tool path (70) for treating the surgical site (S);
during movement of the tool (22) along the tool path (70), detecting (104) a reorientation of the tool (22) and recording a location at which the reorientation occurred; and
generating (106) one or more virtual constraints (VM, SF, PG, FA, FR) that cause the manipulator (14) to reorient (108) the tool (22) in response to the tool (22) revisiting the recorded location.
